# EUROPEAN PATENT APPLICATION

(11) **EP 3 266 874 A1**
(43) Date of publication of application: **10.01.2018**
(21) Application number: 16178606.6
(22) Date of filing: 08.07.2016
(51) Int. Cl.: C12P 13/04, C12P 7/64, C12P 19/02, G01N 33/68, G01N 33/58, G01N 33/92, G01N 24/08

(54) **FERMENTATIVE PRODUCTION AND ISOLATION OF STABLE ISOTOPE LABELED METABOLITES FROM MICROBIAL CELL WALLS AND/OR CELL MEMBRANES**

(71) Applicant: Universität Wien, 1010 Vienna (AT)
(72) Inventor: Hermann, Gerrit, 1180 Wien (AT); Köllensperger, Gunda, 1060 Wien (AT); Rampler, Evelyn, 1150 Wien (AT)
(74) Representative: Vossius & Partner Patentanwälte Rechtsanwälte mbB

(57) **Abstract**

The present invention relates to an improved method for the fermentative production of stable isotope labeled metabolites in a microorganism, particularly stable isotope labeled amino acids, stable isotope labeled monosaccharides, stable isotope labeled lipids, or mixtures thereof, wherein the stable isotope labeled metabolites are isolated from the cell wall and/or the cell membrane of the microorganism. Specifically, the invention provides a method of preparing one or more stable isotope labeled metabolites selected from stable isotope labeled amino acids, stable isotope labeled monosaccharides, stable isotope labeled lipids, and mixtures thereof, the method comprising: cultivating a microorganism in the presence of a stable isotope labeled nutrient; isolating the cell wall and the cell membrane of the microorganism; and isolating said one or more stable isotope labeled metabolites from the isolated cell wall and/or the isolated cell membrane. This method is highly cost-effective and allows to provide the stable isotope labeled metabolite(s) of interest in particularly high concentration and isotopic enrichment. The invention further relates to a composition comprising a mixture of stable isotope labeled metabolites selected from stable isotope labeled amino acids, stable isotope labeled monosaccharides and/or stable isotope labeled lipids, which is obtainable by the aforementioned method, as well as various uses of such a composition, including its use as an internal standard for mass spectrometry or nuclear magnetic resonance (NMR) spectroscopy.

## Description

The present invention relates to an improved method for the fermentative production of stable isotope labeled metabolites in a microorganism, particularly stable isotope labeled amino acids, stable isotope labeled monosaccharides, stable isotope labeled lipids, or mixtures thereof, wherein the stable isotope labeled metabolites are isolated from the cell wall and/or the cell membrane of the microorganism. Specifically, the invention provides a method of preparing one or more stable isotope labeled metabolites selected from stable isotope labeled amino acids, stable isotope labeled monosaccharides, stable isotope labeled lipids, and mixtures thereof, the method comprising: cultivating a microorganism in the presence of a stable isotope labeled nutrient; isolating the cell wall and the cell membrane of the microorganism; and isolating said one or more stable isotope labeled metabolites from the isolated cell wall and/or the isolated cell membrane. This method is highly cost-effective and allows to provide the stable isotope labeled metabolite(s) of interest in particularly high concentration and isotopic enrichment. The invention further relates to a composition comprising a mixture of stable isotope labeled metabolites selected from stable isotope labeled amino acids, stable isotope labeled monosaccharides and/or stable isotope labeled lipids, which is obtainable by the aforementioned method, as well as various uses of such a composition, including its use as an internal standard for mass spectrometry or nuclear magnetic resonance (NMR) spectroscopy.

An internal standard is a chemical substance that is added to a sample to be analyzed, typically at the beginning of the sample preparation and preferably in a defined known concentration, in order to facilitate the qualitative identification and/or the quantitative determination of a corresponding analyte in the sample by an analytical method such as, e.g., mass spectrometry or NMR spectroscopy. The internal standard should ideally have a very high analogy, particularly a high structural similarity, to the corresponding target analyte to be detected in order to ensure that any effects of sample preparation and signal measurement affect both substances in the same manner. By simultaneously detecting the signals of both the internal standard and the corresponding analyte in the sample and correlating them to a calibration curve established with the same concentration of the internal standard and different known concentrations of the corresponding analyte, it is possible to quantitate the amount of analyte contained in the sample. In particular, an external calibration as well as the sample(s) of interest are typically spiked with the same amount of internal standard (volumetric or gravimetric). As the signal of the target compound and the signal of the internal standard are evenly influenced by matrix effects and saturation of the detector, the ratio of both signals will yield a linear calibration function. The principles of isotope dilution guarantee a compensation of the error sources. This leads to an improvement in the linearity of the measurement signal and thus a significant improvement of the results.

The labeling/enrichment of a compound with a stable isotope, such as ¹³C, provides the greatest structural similarity and therefore the closest analogy to the corresponding unlabeled compound/analyte since the stable isotope labeled compound has a different molecular weight than the corresponding (unlabeled) analyte to be detected but is otherwise chemically identical. This makes a stable isotope labeled compound an ideal internal standard, particularly for mass spectrometry or NMR spectroscopy.

Indeed, there is a high demand for stable isotope labeled amino acids, stable isotope labeled monosaccharides and stable isotope labeled lipids to be used as internal standards. In particular, lipids are complex biomolecules that play an essential role in many different biological pathways, maintaining important functions in the human body such as energy storage, signaling and cell wall integrity. Understanding and controlling lipid synthesis and interaction is crucial for the therapeutic intervention of related diseases ranging from inflammation and cancer to metabolic diseases (Wenk MR, Cell, 2010, 143(6):888-95). However, lipid analysis can be hampered by the amphiphilic character of many lipids as well as their biological heterogeneity. Hence, suitable analytical standards, particularly stable isotope labeled standards of biologically relevant lipids, are indispensable.

Stable isotope labeled compounds intended to serve as internal standards, including in particular stable isotope labeled amino acids, monosaccharides and lipids, are conventionally produced by organic synthesis. However, the organic synthesis of stable isotope labeled amino acids, monosaccharides or lipids is typically a protracted procedure and needs dedicated protocols for each compound to be prepared. Besides that, organic chemical syntheses often involve the use of large amounts of toxic substances and environmentally harmful reactants (Sprecher H, Prog Chem Fats Other Lipids, 1978, 15(4):219-54).

An alternative to organic synthesis is the *in vivo* synthesis of stable isotope labeled compounds. In this approach an organism is fed with a stable isotope enriched broth, such that the organism integrates the stable isotope in every molecule of its metabolism. Depending on the cultivation strategy an isotope enrichment degree of over 99% can be achieved. As the whole organism is labeled, purification and enrichment is needed if specific compounds are desired as the final product.

Currently, sources of *in vivo* stable isotope labeled amino acids or monosaccharides are the cytosol of cells and the culture supernatant, which need to be cleaned up by downstream processes. Such processes are costly and inefficient as amino acids and monosaccharides constitute only a small fraction the cytosol or the culture supernatant. Similarly, *in vivo* synthesized, stable isotope labeled lipids can be obtained from whole cell preparations but such preparations require further laborious processing steps in order to provide significant amounts of the labeled lipids of interest. Corresponding methods for the *in vivo* synthesis of stable isotope labeled compounds are described, e.g., in: Neubauer S et al., J Sep Sci, 2012, 35(22):3091-105; Wu L et al., Anal Biochem, 2005, 336(2):164-71; Gevaert K et al., Proteomics, 2008, 8(23-24):4873-85; de Ghellinck A et al., PLoS One, 2014, 9(4):e92999; and WO 2006/105563.

There is, consequently, a need for improved methods for the preparation of stable isotope labeled amino acids, monosaccharides and lipids, which overcome the above-discussed drawbacks and allow the preparation of such stable isotope labeled compounds in a less costly and laborious manner. In particular, there is an urgent need for a simpler and more cost-effective method of preparing stable isotope labeled lipids that can be used as internal standards for mass spectrometry or NMR spectroscopy, given that the limited number and high costs of currently available stable isotope labeled lipids significantly hampers the simultaneous quantification of large numbers of lipids as required, e.g., in lipidomic approaches (Yang K et al., Metabolites, 2011, 1(1):21-40; Han X, "Lipidomics: Comprehensive Mass Spectrometry of Lipids", John Wiley & Sons, 2016).

In the context of the present invention, it was surprisingly found that stable isotope labeled amino acids, stable isotope labeled monosaccharides and/or stable isotope labeled lipids can be provided in particularly high concentrations and purity, with a stable isotope enrichment of >99 atom-%, and in a highly cost-effective manner by cultivating a microorganism in the presence of a stable isotope labeled nutrient and isolating the isotope labeled amino acid(s), the stable isotope labeled monosaccharide(s) and/or the stable isotope labeled lipid(s) of interest specifically from the cell wall and/or the cell membrane of the microorganism. Thus, peptides and proteins as a source of amino acids, polysaccharides (such as, e.g., mannan, glucan, chitin and/or glycans) as a source of monosaccharides as well as lipids can be obtained from the isolated cell wall and/or the isolated cell membrane of the microorganism in considerably greater amounts and in better purity than from the cytosol, from the cell culture supernatant or from whole cell preparations. In this connection, it was particularly unexpected that stable isotope labeled amino acids, stable isotope labeled monosaccharides and stable isotope labeled lipids could be obtained in high isotopic enrichment from the isolated cell wall and/or the isolated cell membrane of the microorganism. The method according to the invention is furthermore advantageous in that it allows the preparation of compositions comprising stable isotope labeled amino acid(s), stable isotope labeled monosaccharide(s) and/or stable isotope labeled lipid(s) having a particularly low content of salts (such as, e.g., Na⁺ and K⁺) as compared to cytosolic preparations or whole cell preparations, which renders these compositions provided in accordance with the invention highly advantageous for the use as internal standards for mass spectrometry. Moreover, a further important advantage of the method according to the invention is the possibility of simultaneously producing, in a short period of time, a set of stable isotope labeled amino acids, monosaccharides and/or lipids *in vivo,* which correspond to naturally occurring biomolecules. In the case of stable isotope labeled lipids, this method does, in fact, constitute the first successful attempt to produce stable isotope labeled versions of naturally occurring lipids by *in vivo* synthesis in a cost-effective manner.

The present invention thus solves the problem of providing an improved method for the preparation of stable isotope labeled metabolites, particularly stable isotope labeled amino acids, stable isotope labeled monosaccharides, stable isotope labeled lipids, and mixtures thereof, which is highly cost-effective and yields the stable isotope labeled metabolite(s) of interest in an advantageously high concentration and isotopic enrichment.

Accordingly, the present invention provides a method of preparing one or more stable isotope labeled metabolites selected from stable isotope labeled amino acids, stable isotope labeled monosaccharides, stable isotope labeled lipids, and mixtures thereof, the method comprising:
- cultivating a microorganism in the presence of a stable isotope labeled nutrient;
- isolating the cell wall and the cell membrane of the microorganism; and
- isolating said one or more stable isotope labeled metabolites from the isolated cell wall and/or the isolated cell membrane.

In a first embodiment of this method, the one or more stable isotope labeled metabolites are one or more stable isotope labeled amino acids and/or one or more stable isotope labeled monosaccharides, and the step of isolating the one or more stable isotope labeled metabolites from the isolated cell wall and/or the isolated cell membrane is preferably conducted by:
- extracting peptides/proteins and/or polysaccharides from the isolated cell wall and/or the isolated cell membrane;
- hydrolyzing said peptides/proteins and/or said polysaccharides and thereby obtaining a mixture of stable isotope labeled amino acids and/or stable isotope labeled monosaccharides; and
- optionally, isolating one or more stable isotope labeled amino acids and/or one or more stable isotope labeled monosaccharides from said mixture.

In accordance with this first embodiment, the invention thus provides a method of preparing one or more stable isotope labeled amino acids and/or one or more stable isotope labeled monosaccharides, the method comprising:
- cultivating a microorganism in the presence of a stable isotope labeled nutrient;
- isolating the cell wall and the cell membrane of the microorganism;
- extracting peptides/proteins and/or polysaccharides from the isolated cell wall and/or the isolated cell membrane;
- hydrolyzing said peptides/proteins and/or said polysaccharides and thereby obtaining a mixture of stable isotope labeled amino acids and/or stable isotope labeled monosaccharides; and
- optionally, isolating one or more stable isotope labeled amino acids and/or one or more stable isotope labeled monosaccharides from said mixture.

In a second embodiment of the method of preparing one or more stable isotope labeled metabolites according to the present invention, the one or more stable isotope labeled metabolites are one or more stable isotope labeled amino acids, and the step of isolating the one or more stable isotope labeled metabolites from the isolated cell wall and/or the isolated cell membrane is preferably conducted by:
- extracting peptides and/or proteins from the isolated cell wall and/or the isolated cell membrane;
- hydrolyzing said peptides and/or proteins and thereby obtaining a mixture of stable isotope labeled amino acids; and
- optionally, isolating one or more stable isotope labeled amino acids from said mixture.

The invention thus also relates to a method of preparing one or more stable isotope labeled amino acids, the method comprising:
- cultivating a microorganism in the presence of a stable isotope labeled nutrient;
- isolating the cell wall and the cell membrane of the microorganism;
- extracting peptides and/or proteins from the isolated cell wall and/or the isolated cell membrane;
- hydrolyzing said peptides and/or proteins and thereby obtaining a mixture of stable isotope labeled amino acids; and
- optionally, isolating one or more stable isotope labeled amino acids from said mixture.

In a third embodiment of the method of preparing one or more stable isotope labeled metabolites according to the invention, the one or more stable isotope labeled metabolites are one or more stable isotope labeled monosaccharides, and the step of isolating the one or more stable isotope labeled metabolites from the isolated cell wall and/or the isolated cell membrane is preferably conducted by:
- extracting polysaccharides from the isolated cell wall and/or the isolated cell membrane;
- hydrolyzing said polysaccharides and thereby obtaining a mixture of stable isotope labeled monosaccharides; and
- optionally, isolating one or more stable isotope labeled monosaccharides from said mixture.

Accordingly, the invention also provides a method of preparing one or more stable isotope labeled monosaccharides, the method comprising:
- cultivating a microorganism in the presence of a stable isotope labeled nutrient;
- isolating the cell wall and the cell membrane of the microorganism;
- extracting polysaccharides from the isolated cell wall and/or the isolated cell membrane;
- hydrolyzing said polysaccharides and thereby obtaining a mixture of stable isotope labeled monosaccharides; and
- optionally, isolating one or more stable isotope labeled monosaccharides from said mixture.

In a fourth embodiment of the method according to the invention, the one or more stable isotope labeled metabolites are a mixture of one or more stable isotope labeled amino acids and one or more stable isotope labeled monosaccharides, and the step of isolating the one or more stable isotope labeled metabolites from the isolated cell wall and/or the isolated cell membrane is preferably conducted by:
- extracting peptides/proteins and polysaccharides from the isolated cell wall and/or the isolated cell membrane;
- hydrolyzing said peptides/proteins and said polysaccharides and thereby obtaining a mixture of stable isotope labeled amino acids and stable isotope labeled monosaccharides; and
- optionally, isolating one or more stable isotope labeled amino acids and one or more stable isotope labeled monosaccharides from said mixture.

The invention thus also relates to a method of preparing a mixture of one or more stable isotope labeled amino acids with one or more stable isotope labeled monosaccharides, the method comprising:
- cultivating a microorganism in the presence of a stable isotope labeled nutrient;
- isolating the cell wall and the cell membrane of the microorganism;
- extracting peptides/proteins and polysaccharides from the isolated cell wall and/or the isolated cell membrane;
- hydrolyzing said peptides/proteins and said polysaccharides and thereby obtaining a mixture of stable isotope labeled amino acids and stable isotope labeled monosaccharides; and
- optionally, isolating one or more stable isotope labeled amino acids and one or more stable isotope labeled monosaccharides from said mixture.

In a fifth embodiment of the method of preparing one or more stable isotope labeled metabolites according to the present invention, the one or more stable isotope labeled metabolites are one or more stable isotope labeled lipids, and the step of isolating the one or more stable isotope labeled metabolites from the isolated cell wall and/or the isolated cell membrane is preferably conducted by:
- extracting lipids from the isolated cell wall and/or the isolated cell membrane and thereby obtaining a mixture of stable isotope labeled lipids; and
- optionally, isolating one or more stable isotope labeled lipids from said mixture.

In accordance with this fifth embodiment, the present invention thus provides a method of preparing one or more stable isotope labeled lipids, the method comprising:
- cultivating a microorganism in the presence of a stable isotope labeled nutrient;
- isolating the cell wall and the cell membrane of the microorganism;
- extracting lipids from the isolated cell wall and/or the isolated cell membrane and thereby obtaining a mixture of stable isotope labeled lipids; and
- optionally, isolating one or more stable isotope labeled lipids from said mixture.

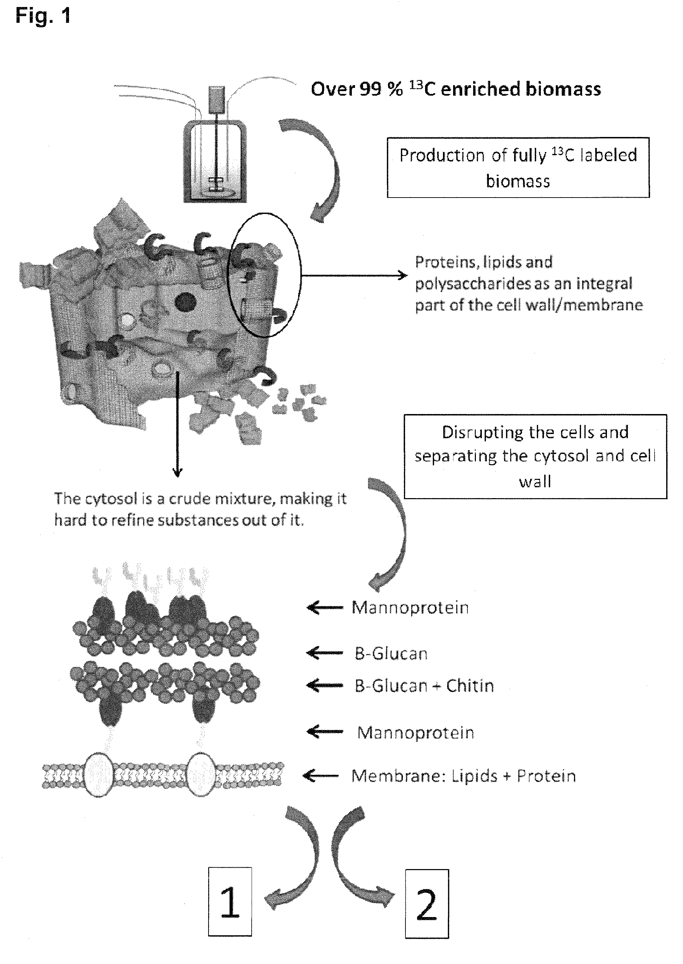

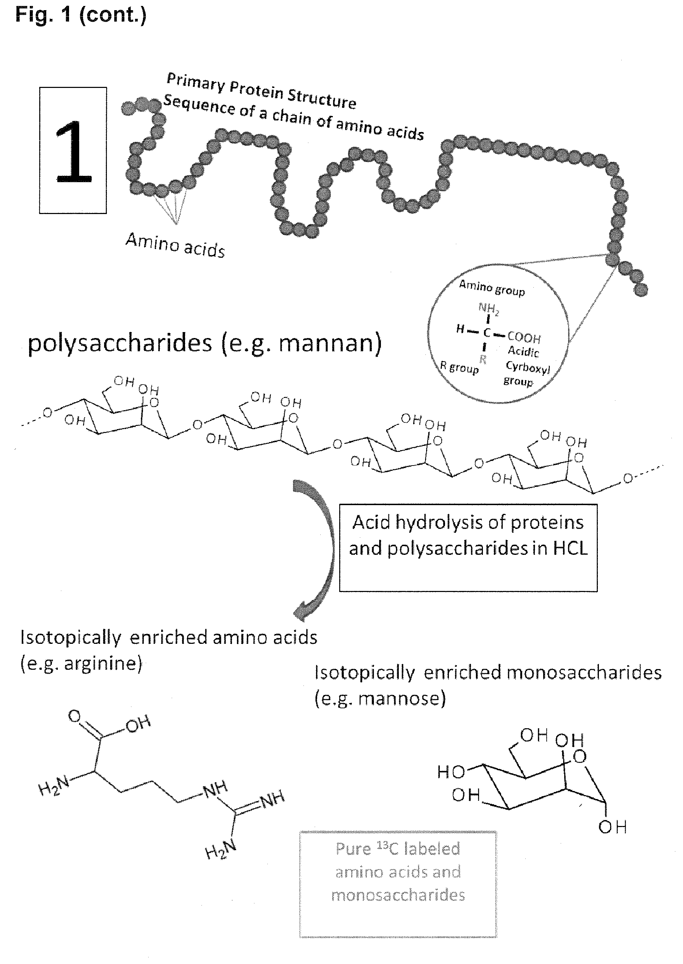

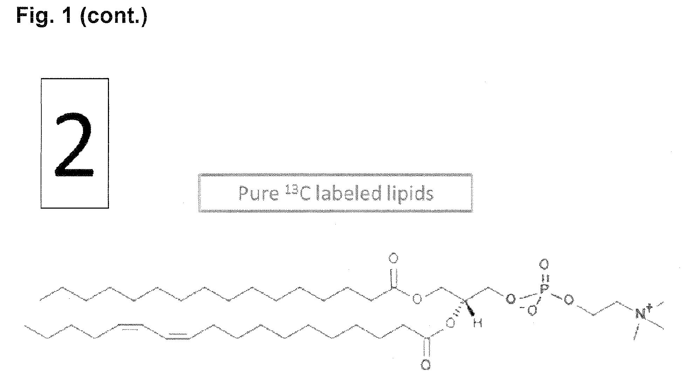

The method of preparing one or more stable isotope labeled metabolites according to the invention is illustrated in Figure 1.

The present invention furthermore provides a composition comprising a mixture of stable isotope labeled metabolites selected from stable isotope labeled amino acids, stable isotope labeled monosaccharides and/or stable isotope labeled lipids, which is obtainable by the method described herein above, including any one of the methods according to the above-described first, second, third, fourth and fifth embodiments.

The invention also relates to various uses of the above-described composition, particularly the use of this composition as an internal standard for mass spectrometry, the use of this composition as an internal standard for nuclear magnetic resonance (NMR) spectroscopy, the use of this composition as a metabolic tracer, or the use of this composition as an ingredient for a culture medium.

The following description of general and preferred features relates to each one of the methods, compositions and uses provided in the present specification, including in particular the methods according to the above-described first, second, third, fourth and fifth embodiments as well as the corresponding compositions obtainable by these methods and the uses of these compositions, unless explicitly indicated otherwise.

The stable isotope serving as a label in the method of preparing one or more stable isotope labeled metabolites in accordance with the present invention is not particularly limited and may be any stable isotope other than the most abundant naturally occurring isotope of the corresponding element. For example, the stable isotope may be selected from ¹³C, ¹⁵N, ¹⁷O, ¹⁸O, ³³S, ³⁴S and any combination thereof. While deuterium (²H or D) can, in principle, also be used as a stable isotope label, it is less suitable for this purpose due to its susceptibility to spontaneous ¹H/D-exchanges in water or aqueous solutions and also due to its effects on the kinetics of chemical or metabolic reactions, which may affect the metabolic profile of a microorganism, e.g., by slowing down the enzymatic desaturation of fatty acids (de Ghellinck A et al., PLoS One, 2014, 9(4):e92999). It is therefore preferred that the stable isotope is not deuterium (²H), i.e. that it is different from deuterium. Preferably, the stable isotope is selected from ¹³C, ¹⁵N, ¹⁸O, ³⁴S, and any combination thereof, and more preferably it is selected from ¹³C, ¹⁵N, ³⁴S, and any combination thereof. Even more preferably, the stable isotope is ¹³C, optionally in combination with ¹⁵N and/or ³⁴S, and most preferably the stable isotope is ¹³C.

In particular, the stable isotope may be a single stable isotope which is preferably selected from ¹³C, ¹⁵N, ¹⁷O, ¹⁸O, ³³S, and ³⁴S, more preferably from ¹³C, ¹⁵N, ¹⁸O, and ³⁴S, even more preferably from ¹³C, ¹⁵N, and ³⁴S, and which is most preferably ¹³C. Alternatively, the stable isotope may be a combination of two or more stable isotopes which are preferably each selected from ¹³C, ¹⁵N, ¹⁷O, ¹⁸O, ³³S, and ³⁴S, more preferably they are each selected from ¹³C, ¹⁵N, ¹⁸O, and ³⁴S, even more preferably they are each selected from ¹³C, ¹⁵N, and ³⁴S, and still more preferably the stable isotope is ¹³C in combination with ¹⁵N and/or ³⁴S; exemplary combinations of two or more stable isotopes include, in particular, ¹³C/¹⁵N, ¹³C/³⁴S, ¹³C/¹⁵N/¹⁸O, or ¹³C/¹⁵N/¹⁸O/³⁴S. Such combinations of two or more stable isotopes are typically combinations of two or more stable isotopes of different elements, such as ¹³C and ¹⁵N, but not combinations of two or more stable isotopes (which are different from the naturally occurring most abundant isotope) of the same element, such as ¹⁷O and ¹⁸O. It is preferred that the stable isotope is a single stable isotope, as described in detail herein above, and most preferably it is ¹³C.

The stable isotope comprised in the one or more stable isotope labeled metabolites to be prepared in the method according to the present invention originates from the stable isotope labeled nutrient, in the presence of which the microorganism is cultivated. Depending on which stable isotope (or combination of stable isotopes) is desired, a corresponding stable isotope labeled nutrient can be suitably chosen. For example, a stable isotope labeled sugar (e.g., a monosaccharide such as glucose or fructose, an oligosaccharide such as lactose or sucrose, a polysaccharide such as starch or cellulose, or any mixture thereof, wherein each of these sugars is labeled (i.e., enriched) with the stable isotope and preferably has the D-configuration) can be used as stable isotope labeled nutrient if the stable isotope to be introduced is a stable isotope of carbon (¹³C), a stable isotope of oxygen (¹⁷O or ¹⁸O), or a combination thereof; the use of a stable isotope labeled glucose is particularly preferred. Moreover, a stable sulfur isotope-enriched sulfate salt (e.g., an alkali metal sulfate, like sodium sulfate or potassium sulfate), such as ³³S-enriched sodium sulfate or ³⁴S-enriched sodium sulfate, can be used as a stable isotope labeled nutrient in order to introduce the corresponding stable sulfur isotope (³³S or ³⁴S). Likewise, to introduce a stable nitrogen isotope (¹⁵N), a stable nitrogen isotope-enriched ammonium salt (e.g., an ammonium salt of an organic or inorganic acid, particularly ammonium sulfate, ammonium hydrogensulfate, ammonium carbonate, ammonium phosphate, ammonium hydrogenphosphate, ammonium dihydrogenphosphate, ammonium formate, ammonium acetate, ammonium oxalate, ammonium fluoride, or ammonium chloride) can be used, such as, e.g., an ¹⁵N-enriched ammonium salt like ¹⁵N-enriched ammonium sulfate. The stable isotope labeled nutrient can also be a combination of two or more stable isotope labeled nutrients which are each labeled with a different stable isotope, including two or more of the above-described examples of stable isotope labeled nutrients, in order to provide metabolites that are labeled with two or more different stable isotopes.

It is preferred that the stable isotope labeled nutrient is uniformly labeled with the stable isotope, i.e. that the stable isotope replaces all atoms of the corresponding element comprised in the stable isotope labeled nutrient. For example, if ¹³C-labeled glucose is used as the stable isotope labeled nutrient, then it is preferred to use uniformly ¹³C-labeled glucose, in which all six carbon atoms of glucose are present as ¹³C atoms (i.e., ¹³C₆-glucose). Furthermore, it is preferred that the stable isotope labeled nutrient is the sole source of the corresponding element, in the presence of which the microorganism is cultivated. Accordingly, it is particularly preferred that the stable isotope uniformly labels all nutrients that constitute sources of the corresponding element and in the presence of which the microorganism is cultivated.

It is further preferred that the stable isotope labeled nutrient (which is preferably uniformly labeled with the stable isotope) has an isotope enrichment of at least about 80 atom-%, more preferably at least about 90 atom-%, even more preferably at least about 95 atom-%, yet even more preferably at least about 98 atom-%, yet even more preferably at least about 99 atom-%, and still more preferably at least about 99.5 atom-%. If a stable isotope labeled nutrient that is labeled with stable isotopes of two or more different elements is used, or if two or more stable isotope labeled nutrients which are each labeled with a stable isotope of a different element are used, it is preferred that the aforementioned isotope enrichment values apply to each of the different stable isotopes.

It is particularly preferred that the stable isotope is ¹³C, that the stable isotope labeled nutrient is ¹³C-labeled glucose, and that the ¹³C-labeled glucose is the sole carbon source in the presence of which the microorganism is cultivated. The ¹³C-labeled glucose is preferably uniformly ¹³C-labeled glucose and/or it preferably has an isotope enrichment of at least about 90 atom-%, more preferably at least about 95 atom-%, even more preferably at least about 98 atom-%, yet even more preferably at least about 99 atom-%, and still more preferably at least about 99.5 atom-%. Accordingly, it is particularly preferred that the microorganism is cultivated in a culture medium containing uniformly ¹³C-labeled glucose having an isotope enrichment of at least about 90 atom-% (more preferably at least about 95 atom-%, even more preferably at least about 98 atom-%, yet even more preferably at least about 99 atom-%, and still more preferably at least about 99.5 atom-%) as the sole carbon source.

The one or more stable isotope labeled metabolites to be prepared in the method of the present invention preferably are uniformly labeled with the stable isotope. Accordingly, it is preferred that the stable isotope labeled amino acid(s), the stable isotope labeled monosaccharide(s) and/or the stable isotope labeled lipid(s) is/are uniformly labeled with the stable isotope.

Moreover, it is preferred that the one or more stable isotope labeled metabolites (which are preferably uniformly labeled with the stable isotope) have an isotope enrichment of at least about 70 atom-%, more preferably at least about 80 atom-%, even more preferably at least about 90 atom-%, yet even more preferably at least about 95 atom-%, yet even more preferably at least about 98 atom-%, yet even more preferably at least about 99 atom-%, and still more preferably at least about 99.5 atom-%. If the one or more stable isotope labeled metabolites contain two or more different stable isotopes (of different elements), it is preferred that the aforementioned isotope enrichment values apply to each one of the two or more different stable isotopes. The isotope enrichment in respect of a specific stable isotope in a corresponding stable isotope labeled metabolite can be determined using techniques known in the art, such as, e.g., mass spectrometry or NMR spectroscopy.

It is particularly preferred that the stable isotope is ¹³C, and that the one or more stable isotope labeled metabolites are uniformly ¹³C-labeled metabolites having an isotope enrichment of at least about 90 atom-% (more preferably at least about 95 atom-%, even more preferably at least about 98 atom-%, yet even more preferably at least about 99 atom-%, and still more preferably at least about 99.5 atom-%).

The microorganism to be used in the method of the present invention is not particularly limited and may be any microorganism that has a cell wall and is capable of being cultivated in a culture medium, including, e.g., bacterial cells, fungal cells, or plant cells. In general, the microorganism and the corresponding culturing conditions should preferably be selected so as to ensure that the microorganism does not fix/incorporate carbon or oxygen from the surrounding atmosphere if the stable isotope to be incorporated is a stable isotope of carbon or oxygen, or the corresponding gases, such as CO₂, should be removed from the surrounding atmosphere and/or from the fermenter inlet gas during the culturing, e.g., by using a CO₂ remover/scrubber. It is preferred that the microorganism to be used in accordance with the invention is a fungus (particularly a yeast) or a bacterium. Examples of a bacterium to be used in the method of the invention include, in particular, a bacterium of the genus *Escherichia* (e.g. *Escherichia coli*)*, Zymomonas* (e.g., *Zymomonas mobilis*), *Rhodococcus* (e.g., *Rhodococcus erythropolis*), *Pseudomonas* (e.g., *Pseudomonas putida*), *Bacillus* (e.g., *Bacillus licheniformis* or *Bacillus subtilis), Lactobacillus* (e.g., *Lactobacillus plantarum), Enterococcus* (e.g., *Enterococcus faecium, Enterococcus gallinarium,* or *Enterococcus faecalis), Pediococcus* (e.g., *Pediococcus pentosaceus* or *Pediococcus acidilactici*), *Alcaligenes* (e.g. *Alcaligenes eutrophus*), *Klebsiella, Paenibacillus* (e.g., *Paenibacillus macerans*), *Arthrobacter, Corynebacterium* (e.g., *Corynebacterium glutamicum),* or *Brevibacterium,* and a preferred example is *Escherichia coli.* More preferably, the microorganism to be used in the method of the present invention is a yeast, and even more preferably a yeast selected from the genus *Pichia* (e.g., *Pichia pastoris*), *Saccharomyces* (e.g., *Saccharomyces cerevisiae), Candida* (e.g., *Candida albicans*), *Hansenula* (e.g., *Hansenula polymorpha), Schizosaccharomyces* (e.g., *Schizosaccharomyces pombe),* or *Kluyveromyces* (e.g., *Kluyveromyces marxianus* or *Kluyveromyces lactis*). Still more preferably, the microorganism to be used in the method of the present invention is a yeast of the genus *Pichia,* and most preferably it is *Pichia pastoris* (e.g., *P. pastoris* AJ 5076, *P. pastoris* ATCC 28485, *P. pastoris* ATCC 3650, *P. pastoris* BCRC 21531, *P. pastoris* CBS 704, *P. pastoris* CBS 7435, *P. pastoris* CCRC 21531, *P. pastoris* CCT 2587, *P. pastoris* CCT 2644, *P. pastoris* CCY 21-43-1, *P. pastoris* CCY 39-29-2, *P. pastoris* CECT 11078, *P. pastoris* CECT 11484, *P. pastoris* CLIB 289, *P. pastoris* DSM 70382, P. *pastoris* DSMZ 70382, *P. pastoris* GS 115, *P. pastoris* HUT-7288, *P. pastoris* IAM 12903, P. *pastoris* IFO 10777, *P. pastoris* JCM 3650, *P. pastoris* KCTC 7190, *P. pastoris* LY 80, P. *pastoris* MG 65, *P. pastoris* MUCL 27793, *P. pastoris* MUCL 31260, *P. pastoris* NBRC 10777, *P. pastoris* NCAIM Y.01243, *P. pastoris* NCIM 3490, *P. pastoris* NCTC 2058, *P. pastoris* NCYC 175, *P. pastoris* NRRL Y-1603, *P. pastoris* OUT 20010, *P. pastoris* VKM Y-1379, P. *pastoris* VKM Y-1623, *P. pastoris VKM* Y-884, *P. pastoris* BG09, *P. pastoris* BG10, *P. pastoris* BG11, *P. pastoris* BG12, or *P. pastoris* SuperMan₅). Yeasts that are Crabtree effect negative, such as *Pichia pastoris,* allow for a higher conversion rate from sugar to biomass and are therefore economically preferable over yeasts that are Crabtree effect positive, such as *Saccharomyces cerevisiae;* Crabtree effect positive yeasts are capable of converting glucose directly to ethanol, which is unfavorable as it results in a lower sugar to biomass conversion. Furthermore, the use of *Pichia pastoris* is particularly advantageous as this yeast is not only growing on ¹³C-labeled glucose as sole carbon source and offering a similar lipid profile to other higher eukaryotes, but can also be fermented to very high cell densities, thereby increasing product yields, and has a particular stable cell wall, so that long-term storage and reuse is simplified.

The microorganism can be cultivated in a culture medium in a fermenter, wherein the culture medium contains the stable isotope labeled nutrient, at a suitable temperature depending on the requirements of the particular microorganism. The temperature of the culture medium may be, e.g., about 20°C to about 37°C, is preferably about 25°C to about 32°C, and is more preferably about 27°C, particularly if the microorganism is a yeast (e.g., a yeast of the genus *Pichia,* such as *Pichia pastoris).* If the culture medium contains a ¹³C-labeled sugar (preferably ¹³C-labeled glucose) as the sole carbon source, it may be present at a concentration of, e.g., about 1 g/L to about 20 g/L, particularly about 5 g/L to about 15 g/L (e.g., about 10 g/L). A corresponding ¹³C-labeled sugar may also be employed at higher concentrations but this will typically not further increase the efficiency of the process. In addition to an appropriate carbon source, the culture medium typically also contains suitable minerals, salts, cofactors, buffers and/or other components, known to a person skilled in the art, which are suitable for the growth of the microorganism. The fermenter which may be used for cultivating the microorganism is not particularly limited and may be, e.g., a stirred tank fermenter, an airlift fermenter, a bubble column fermenter, or any combination thereof. Suitable conditions for cultivating a yeast such as *Pichia pastoris* are furthermore described, e.g., in Neubauer S et al., J Sep Sci, 2012, 35(22):3081-105.

The method of preparing one or more stable isotope labeled metabolites may comprise, after the step of cultivating the microorganism and prior to the step of isolating the cell wall and the cell membrane of the microorganism, a further step of quenching the cells of the microorganism, e.g., by subjecting the cells of the microorganism to cold methanol quenching. This optional quenching step is preferably conducted using methanol (e.g., pure methanol or a mixture of methanol and water) at a temperature of about -15°C to about -45°C, more preferably at a temperature of about -20°C to about -40°C, even more preferably at a temperature of about -25°C to about -35°C, and still more preferably at a temperature of about -30°C. Accordingly, it is preferred that this step is conducted by subjecting the cells of the microorganism to methanol (e.g., pure methanol or a mixture of methanol and water) at a temperature of about -15°C to about -45°C, more preferably at a temperature of about -20°C to about -40°C, even more preferably at a temperature of about -25°C to about -35°C, and still more preferably at a temperature of about -30°C. Quenching agents other than cold methanol can also be used, such as cold ethanol (e.g., pure ethanol or a mixture of ethanol and water), cold propanol (e.g., pure propanol or a mixture of propanol and water), or cold glycerol-saline (e.g., as described in Villas-Bôas SG et al., Anal Biochem, 2007, 370(1):87-97).

The method of preparing one or more stable isotope labeled metabolites according to the present invention comprises a step of isolating the cell wall and the cell membrane of the microorganism. This step is preferably conducted by boiling the cells of the microorganism in an ethanol/water mixture and filtering off the cell wall and the cell membrane. Accordingly, it is preferred that the cells of the microorganism are incubated in a boiling ethanol/water mixture (i.e., an ethanol/water mixture heated to boiling) to open the cell wall and the cell membrane of the microorganism, and that the cell wall and the cell membrane of the microorganism are filtered off to separate them from other cellular components of the microorganism, particularly from cytosolic components. The ethanol/water mixture preferably contains (or, more preferably, consists of) ethanol and water in a ratio of about 50-100% (v/v) to about 50-0% (v/v), particularly in a ratio of ethanol to water of about 60-90% (v/v) to about 40-10% (v/v), even more preferably in a ratio of ethanol to water of about 70-80% (v/v) to about 30-20% (v/v), and still more preferably in a ratio of ethanol to water of about 75% (v/v) to about 25% (v/v). It will be understood that the aforementioned ratios defining the relative volumes of ethanol and water in the ethanol/water mixture may change in the course of the boiling treatment. After boiling the cells of the microorganism in the ethanol/water mixture, this mixture is subjected to a filtering step to separate the cell wall and the cell membrane of the microorganism from other cellular components, particularly from cytosolic components. While the present invention also encompasses the isolation of either the cell wall or the cell membrane of the microorganism, typically both the cell wall (or fragments thereof) and the cell membrane (or fragments thereof) will be retained in the filter and can be harvested, e.g., by scratching them from the filter. The cell wall and the cell membrane can be obtained from the filter in solid form, which makes this procedure particularly efficient and easy to conduct. The filter preferably has a pore size of about 0.5 µm to about 2 µm, more preferably of about 1 µm. A filter having a smaller pore size is advantageous in that it also holds back smaller fragments of the cell wall and/or the cell membrane. The material of the filter is not particularly limited. Alternatively, the step of isolating the cell wall and the cell membrane of the microorganism can also be conducted as described above but using a boiling methanol/water mixture or a boiling propanol/water mixture (or any other extraction solution or mixture of solvents that is suitable for extracting polar and non-polar substances) in place of the above-discussed boiling ethanol/water mixture. As a further alternative, the step of isolating the cell wall and the cell membrane of the microorganism can be conducted by subjecting the cells of the microorganism to a freeze-thaw treatment (e.g., to multiple freeze-thaw cycles), and/or by subjecting the cells of the microorganism to sonication (e.g., by applying sound energy at a frequency equal to or greater than about 16 kHz, which is also referred to as "ultrasound", particularly at a frequency from about 16 kHz to about 200 MHz, preferably from about 20 kHz to about 2 MHz, more preferably from about 25 kHz to about 200 kHz, and even more preferably from about 30 kHz to about 100 kHz), and/or by treating the cells of the microorganism with a detergent (e.g., a detergent selected from sodium dodecyl sulfate (SDS), octylphenoxypolyethoxyethanol (e.g., Nonidet P-40 or IGEPAL CA-630), Triton X-100, n-dodecyl-β-D-maltopyranoside (DDM), digitonin, Polysorbate 20 (e.g., Tween 20), Polysorbate 80 (e.g., Tween 80), 3-[(3-cholamidopropyl)dimethylammonio]-1-propanesulfonate (CHAPS), urea, cholate, sodium lauroyl sarcosinate (sarkosyl), and any combination thereof; preferably an SDS-based aqueous buffer (such as: 0.5% (w/v) SDS, 0.05 M Tris-Cl, 1 mM dithiothreitol (DTT), pH 8.0), an octylphenoxypolyethoxyethanol-based aqueous buffer (such as: 150 mM NaCl, 1.0% (w/v) octylphenoxypolyethoxyethanol, 50 mM Tris-Cl, pH 7.4), or a Triton X-100-based aqueous buffer (such as: 0.1% (w/v) Triton X-100)); in all these cases, the cell wall and the cell membrane of the microorganism can then be filtered off to separate them from other cellular components (particularly cytosolic components) of the microorganism, as described above. In any of the above-mentioned methods for isolating the cell wall and the cell membrane of the microorganism, it is also possible to use centrifugation instead of (or in addition to) filtration in order to separate the cell wall and the cell membrane from other cellular components of the microorganism, particularly from cytosolic components. Accordingly, the step of isolating the cell wall and the cell membrane of the microorganism may be conducted by: (i) boiling the cells of the microorganism in an ethanol/water mixture, in a methanol/water mixture or in a propanol/water mixture, and then isolating the cell wall and the cell membrane by filtration and/or centrifugation; or (ii) subjecting the cells of the microorganism to a freeze-thaw treatment, and then isolating the cell wall and the cell membrane by filtration and/or centrifugation; or (iii) subjecting the cells of the microorganism to sonication, and then isolating the cell wall and the cell membrane by filtration and/or centrifugation; or (iv) treating the cells of the microorganism with a detergent, and then isolating the cell wall and the cell membrane by filtration and/or centrifugation. It will be understood that any one of the methods for isolating the cell wall and the cell membrane of the microorganism described herein can be used alone, or two or more of these methods can be used in combination.

As described above, the method according to the present invention may comprise, after the step of cultivating the microorganism, a step of quenching the cells of the microorganism and a subsequent step of isolating the cell wall and the cell membrane of the microorganism. These latter two steps may, for example, be conducted as follows: The cells of the microorganism are first harvested directly into the cooled quenching solvent (which may, in principle, be any mixture of methanol/water or ethanol/water or propanol/water, and which is preferably methanol at a temperature as described further above), and are then subjected to centrifugation (preferably in a cooled centrifuge) to separate the cells from the quenching solvent. When the quenching solvent and the cells are separated, the cells can be transferred into an extraction solvent, which may be, e.g., any boiling mixture of ethanol/water or methanol/water or propanol/water (and which is preferably a boiling ethanol/water mixture as described above). The resulting reaction mixture can then be subjected to centrifugation or to filtration (or to a combination of both, such as centrifugation followed by filtration, or filtration followed by centrifugation) in order to separate the cell wall and the cell membrane from other cellular components (particularly cytosolic components) of the microorganism.

The isolated cell wall and/or the isolated cell membrane can further be disrupted in order to obtain smaller fragments, which allows to improve the efficiency of the subsequent extraction step. It is thus preferred to subject the isolated cell wall and/or the isolated cell membrane to mechanical disruption (using, e.g., a homogenizer, a ball mill, sonication, and/or a French press). Accordingly, the method of the present invention preferably comprises, after the step of isolating the cell wall and the cell membrane of the microorganism and prior to the step of isolating the one or more stable isotope labeled metabolites from the isolated cell wall and/or the isolated cell membrane, a further step of subjecting the isolated cell wall and the isolated cell membrane to mechanical disruption.

In accordance with the first embodiment of the method according to the present invention, the one or more stable isotope labeled metabolites to be prepared in this method are one or more stable isotope labeled amino acids and/or one or more stable isotope labeled monosaccharides, and the step of isolating the one or more stable isotope labeled metabolites from the isolated cell wall and/or the isolated cell membrane is preferably conducted by:
- extracting peptides/proteins and/or polysaccharides from the isolated cell wall and/or the isolated cell membrane;
- hydrolyzing said peptides/proteins and/or said polysaccharides and thereby obtaining a mixture of stable isotope labeled amino acids and/or stable isotope labeled monosaccharides; and
- optionally, isolating one or more stable isotope labeled amino acids and/or one or more stable isotope labeled monosaccharides from said mixture.

The polysaccharides (such as, e.g., mannan, glucan, chitin, galactomannan, glucomannan, rhamnomannan, phosphomannan, N-glycans or O-glycans) as well as the peptides/proteins can be extracted from the isolated cell wall and/or the isolated cell membrane, e.g., by using an organic solvent, separating the aqueous phase and the organic phase, and retrieving the polysaccharides and/or the peptides/proteins from the aqueous phase and from the intermediate phase (i.e., the boundary between organic and aqueous phase), respectively. Accordingly, the isolated cell wall and/or the isolated cell membrane can be subjected to an organic solvent such as, e.g., methyl-tert-butyl ether, chloroform/methanol, dichloromethane/methanol, hexane, hexane/isopropanol, acetone/ethanol, ethanol/diethyl ether, or diethyl ether. This allows to obtain the polysaccharides in the aqueous phase and in the intermediate phase together with precipitated proteins/peptides (which are only present in the intermediate phase), while the lipids will be present in the organic phase. In this manner, the polysaccharides and the peptides/proteins can be separated efficiently from the lipids. The polysaccharides and the peptides/proteins can then be subjected to hydrolysis to obtain amino acids and monosaccharides, and the amino acids and monosaccharides thus obtained can subsequently be separated from one another. The organic solvent extraction procedures detailed further below in connection with the extraction of lipids from the isolated cell wall and/or the isolated cell membrane can be applied in an analogous manner in order to extract peptides/proteins and/or polysaccharides from the isolated cell wall and/or the isolated cell membrane.

The peptides and/or proteins can be extracted from the isolated cell wall and/or the isolated cell membrane in precipitated form not only by using an organic solvent, as described above, but also using other suitable precipitation reagents such as, e.g., ammonium sulfate. Accordingly, the peptides and/or proteins can be extracted from the isolated cell wall and/or the isolated cell membrane using, e.g., one or more precipitation methods selected from chloroform/methanol precipitation, methyl-tert-butyl ether precipitation, diethyl ether precipitation, acetone precipitation, acidified acetone/methanol precipitation, trichloroacetic acid precipitation, and ammonium sulfate precipitation. In this manner, the peptides and/or proteins can be precipitated and can thereby be separated from other components of the isolated cell wall and/or the isolated cell membrane, particularly from the lipids comprised in the isolated cell wall and/or the isolated cell membrane.

After the extraction of peptides/proteins and/or polysaccharides from the isolated cell wall and/or the isolated cell membrane, the peptides/proteins and/or the polysaccharides are hydrolyzed to obtain a mixture of stable isotope labeled amino acids and/or stable isotope labeled monosaccharides. It will be understood that the hydrolysis of glycopeptides/glycoproteins can yield both stable isotope labeled amino acids and stable isotope labeled monosaccharides. The hydrolysis of the peptides/proteins and/or the polysaccharides is preferably conducted under acidic conditions (i.e., acidic hydrolysis). For example, the peptides/proteins and/or the polysaccharides can be subjected to hydrochloric acid, particularly to 6 M HCl at a temperature of about 100°C to about 110°C, e.g., for a period of about 24 h. Alternatively, the polysaccharides (and/or the peptides/proteins) can also be subjected to trifluoroacetic acid (TFA), e.g. at a temperature of about 105°C to about 125°C; or they can be subjected to sulfuric acid (H₂SO₄), e.g. at room temperature, which is advantageous over TFA hydrolysis as it also allows to obtain N-acetylglucosamine (e.g., from chitin). The polysaccharides can also be subjected to sodium borohydride (NaBH₄) in order to obtain O-glycans (through chemical release by beta elimination). Further methods of hydrolyzing peptides/proteins and/or polysaccharides, including in particular acidic hydrolysis methods, alkaline hydrolysis methods or enzymatic hydrolysis methods, can also be used. Such methods are known in the art and are described, e.g., in: Kellermann J, "Spaltung von Proteinen", in Lottspeich F & Engels WJ (eds.), "Bioanalytik", Third Edition, Springer-Verlag, 2012, pages 230-242. By suitably adjusting the conditions of this hydrolysis step, a partial or complete hydrolysis of the peptides/proteins and/or the polysaccharides from the isolated cell wall and/or the isolated cell membrane can be achieved. If peptides/proteins and polysaccharides have been extracted from the isolated cell wall and/or the isolated cell membrane, it is preferred to simultaneously hydrolyze both the peptides/proteins and the polysaccharides (particularly by acidic hydrolysis, as described above) to obtain a mixture of stable isotope labeled amino acids and stable isotope labeled monosaccharides, which can subsequently be separated (as desired).

Optionally, one or more stable isotope labeled amino acids and/or one or more stable isotope labeled monosaccharides can be isolated from the mixture of stable isotope labeled amino acids and/or stable isotope labeled monosaccharides which is obtained by hydrolyzing the extracted peptides/proteins and/or polysaccharides. Any one or more stable isotope labeled amino acids and/or any one or more stable isotope labeled monosaccharides from said mixture can be isolated, as desired, using for example chromatography, electrophoresis, or other techniques for the separation of amino acids or sugars (e.g., as described in: Boysen R, "Chromatographische Trennmethoden", in Lottspeich F & Engels WJ (eds.), "Bioanalytik", Third Edition, Springer-Verlag, 2012, pages 243-268; Westermeier R et al., "Elektrophoretische Verfahren", in Lottspeich F & Engels WJ (eds.), "Bioanalytik", Third Edition, Springer-Verlag, 2012, pages 269-302; or Schmitt-Kopplin P et al., "Kapillarelektrophorese", in Lottspeich F & Engels WJ (eds.), "Bioanalytik", Third Edition, Springer-Verlag, 2012, pages 303-333). The method according to the first or fourth embodiment thereby allows to obtain any one stable isotope labeled amino acid, or a mixture of two or more (or, e.g., three or more, or five or more, or eight or more, or twelve or more) stable isotope labeled amino acids, which may be selected from any of the exemplary or preferred stable isotope labeled amino acids described further below, and/or any one stable isotope labeled monosaccharide, or a mixture of two or more (or, e.g., three or more, or four or more, or five or more, or six or more) stable isotope labeled monosaccharides, which may be selected from any of the exemplary or preferred stable isotope labeled monosaccharides described further below. Likewise, the method according to the second embodiment thereby allows to obtain any one stable isotope labeled amino acid, or a mixture of two or more (or, e.g., three or more, or five or more, or eight or more, or twelve or more) stable isotope labeled amino acids, which may be selected from any of the exemplary or preferred stable isotope labeled amino acids described further below; and the method according to the third embodiment thus allows to obtain any one stable isotope labeled monosaccharide, or a mixture of two or more (or, e.g., three or more, or four or more, or five or more, or six or more) stable isotope labeled monosaccharides, which may be selected from any of the exemplary or preferred stable isotope labeled monosaccharides described further below.

In accordance with the fifth embodiment of the method according to the invention, the one or more stable isotope labeled metabolites to be prepared in this method are one or more stable isotope labeled lipids, and the step of isolating the one or more stable isotope labeled metabolites from the isolated cell wall and/or the isolated cell membrane is preferably conducted by:
- extracting lipids from the isolated cell wall and/or the isolated cell membrane and thereby obtaining a mixture of stable isotope labeled lipids; and
- optionally, isolating one or more stable isotope labeled lipids from said mixture.

The lipids can be extracted from the isolated cell wall and/or the isolated cell membrane using, e.g., an organic solvent. Accordingly, the isolated cell wall and/or the isolated cell membrane can be subjected to an organic solvent such as, e.g., methyl-tert-butyl ether, chloroform/methanol, dichloromethane/methanol, hexane, hexane/isopropanol, acetone/ethanol, ethanol/diethyl ether, or diethyl ether. Preferably, the organic solvent to be used in this extraction step is methyl-tert-butyl ether (MTBE) or chloroform/methanol (C/M). The extraction of lipids with chloroform/methanol can be performed, for example, as a single-step extraction with 2:1 C/M (e.g., as described in Folch J et al., J Biol Chem, 1957, 226(1):497-509) or as a two-step extraction with 17:1 C/M in the first step and 2:1 C/M in the second step. In particular, the step of extracting lipids from the isolated cell wall and/or the isolated cell membrane can be conducted by suspending the isolated cell wall and/or the isolated cell membrane in chloroform/methanol (e.g., in 2:1 C/M, v/v), isolating the liquid phase, inducing phase separation by adding water or an aqueous solution, and collecting the organic phase containing the lipids. The extraction of lipids with methyl-tert-butyl ether can also be performed, e.g., as described in Matyash V et al., J Lipid Res, 2008, 49(5):1137-46. In particular, the step of extracting lipids from the isolated cell wall and/or the isolated cell membrane can be performed by suspending the isolated cell wall and/or the isolated cell membrane in methanol, adding methyl-tert-butyl ether, inducing phase separation by adding water or an aqueous solution, and collecting the organic phase containing said lipids. Using any of these extraction methods, the lipids contained in the isolated cell wall and/or the isolated cell membrane can be separated from other components of the isolated cell wall and/or the isolated cell membrane, particularly from the peptides/proteins and the polysaccharides comprised in the isolated cell wall and/or the isolated cell membrane, so that a mixture of stable isotope labeled lipids is obtained.

Optionally, one or more stable isotope labeled lipids can be isolated from the mixture of stable isotope labeled lipids thus obtained. Any one or more stable isotope labeled lipids from said mixture can be isolated, as desired, using for example chromatography, solvent fractionation, solid phase extraction or other techniques for the separation of lipids (e.g., as described in Hanahan DJ et al., "Lipide chemistry", John Wiley & Sons, 1960; Cífková E et al., Anal Chem, 2012, 84(22):10064-70; Kaluzny MA et al., J Lipid Res, 1985, 26(1):135-40; Nichols BW et al., Br Med Bull, 1966, 22(2):137-46; Kim HY et al., J Lipid Res, 1990, 31(12):2285-9; or Lottspeich F & Engels WJ (eds.), "Bioanalytik", Third Edition, Springer-Verlag, 2012, and references cited therein). The method according to the fifth embodiment thereby allows to obtain any one stable isotope labeled lipid, or a mixture of two or more (or, e.g., three or more, or five or more, or ten or more, or 20 or more, or 30 or more, or 40 or more, or 50 or more, or 75 or more, or 100 or more) stable isotope labeled lipids, which may be selected from any of the exemplary or preferred stable isotope labeled lipids described further below.

The method of the present invention furthermore allows the preparation of one or more stable isotope labeled lipids (in a first composition) as well as one or more stable isotope labeled amino acids and/or one or more stable isotope labeled monosaccharides (in a second composition). In this case, the step of isolating the one or more stable isotope labeled metabolites from the isolated cell wall and/or the isolated cell membrane can be conducted by subjecting the isolated cell wall and/or the isolated cell membrane to an organic solvent (such as, e.g., methyl-tert-butyl ether, chloroform/methanol, dichloromethane/methanol, hexane, hexane/isopropanol, acetone/ethanol, ethanol/diethyl ether, or diethyl ether, as described above) so that, upon phase separation between the aqueous phase and the organic phase, the lipids are obtained in the organic phase, the proteins/peptides are precipitated in the intermediate phase (between the organic phase and the aqueous phase), and the polysaccharides are obtained in the aqueous phase and in the intermediate phase. The lipids can be extracted from the organic phase to obtain a mixture of stable isotope labeled lipids (first composition; as described in relation to the fifth embodiment), while the polysaccharides and the proteins/peptides can be extracted from the aqueous phase and the intermediate phase and can then be hydrolyzed to obtain a mixture of stable isotope labeled amino acids and stable isotope labeled monosaccharides, which can be further separated to obtain one or more stable isotope labeled amino acids and/or one or more stable isotope labeled monosaccharides (second composition; as described in relation to the first to fourth embodiments). The first composition comprising one or more stable isotope labeled lipids and the second composition comprising one or more stable isotope labeled amino acids and/or one or more stable isotope labeled monosaccharides can be combined, if desired, to obtain a mixture of one or more stable isotope labeled lipids with one or more stable isotope labeled amino acids and/or with one or more stable isotope labeled monosaccharides.

The method of the present invention, in principle, allows the preparation of any stable isotope labeled amino acid, any stable isotope labeled monosaccharide and/or any stable isotope labeled lipid, provided that the corresponding amino acid, monosaccharide or lipid is produced by the microorganism and is incorporated into its cell wall and/or cell membrane. The preparation of a particular stable isotope labeled amino acid, a particular stable isotope labeled monosaccharide or a particular stable isotope labeled lipid (or any particular set of such compounds), as desired, can be facilitated by suitably choosing the microorganism and the culturing conditions such that high amounts of the stable isotope labeled metabolite(s) of interest are incorporated into the cell wall and/or the cell membrane of the microorganism. In particular, the culturing conditions under which the microorganism is cultivated can be varied/adjusted in order to influence the composition of the cell wall of the microorganism, including the composition of peptide/protein components, polysaccharide components and/or lipid components of the cell wall (see, e.g., Killick KA, J Bacteriol, 1971, 106(3):931-7), depending on which amino acid(s), monosaccharide(s) and/or lipid(s) are to be prepared.

The stable isotope labeled amino acid(s) to be prepared by the method of the present invention, including in particular the method according to the first, the second or the fourth embodiment, is/are preferably selected from Ala, Arg, Asn, Asp, Cys, Glu, Gln, Gly, His, ile, Leu, Lys, Met, Phe, Pro, Ser, Thr, Trp, Tyr, and Val, all of which are stable isotope labeled, and all of which preferably have the L-configuration. More preferably, the stable isotope labeled amino acid(s) is/are selected from Ala, Arg, Asp, Cys, Glu, Gly, His, ile, Leu, Lys, Met, Phe, Pro, Ser, Thr, Trp, Tyr, and Val, all of which are stable isotope labeled, and all of which preferably have the L-configuration. Even more preferably, the stable isotope labeled amino acid(s) is/are selected from Ala, Arg, Asp, Glu, Gly, His, ile, Leu, Lys, Phe, Pro, Ser, Thr, Tyr, and Val, all of which are stable isotope labeled, and all of which preferably have the L-configuration. Yet even more preferably, the stable isotope labeled amino acid(s) is/are selected from Ala, Arg, Asp, Glu, Gly, His, ile, Leu, Lys, Phe, Pro, Ser, Thr, Tyr, and Val, all of which are uniformly stable isotope labeled (e.g., uniformly ¹³C-labeled), and all of which preferably have the L-configuration. Examples of stable isotope labeled amino acid(s) to be prepared by the method of the present invention include, in particular, L-Ala (U-¹³C) (i.e., uniformly ¹³C-labeled L-alanine), L-Ala (¹⁵N), L-Ala (U-¹³C, ¹⁵N), L-Ala (U-¹⁸O), L-Ala (U-¹³C, U-¹⁸O), L-Arg (U-¹³C), L-Arg (U-¹⁵N), L-Arg (U-¹³C, U-¹⁵N), L-Arg (U-¹⁸O), L-Arg (U-¹³C, U-¹⁸O), L-Asn (U-¹³C), L-Asn (U-¹⁵N), L-Asn (U-¹³C, U-¹⁵N), L-Asn (U-¹⁸O), L-Asn (U-¹³C, U-¹⁸O), L-Asp (U-¹³C), L-Asp (U-¹⁵N), L-Asp (U-¹³C, U-¹⁵N), L-Asp (U-¹⁸O), L-Asp (U-¹³C, U-¹⁸O), L-Cys (U-¹³C), L-Cys (¹⁵N), L-Cys (U-¹³C, ¹⁵N), L-Cys (U-¹⁸O), L-Cys (U-¹³C, U-¹⁸O), L-Cys (³⁴S), L-Cys (U-¹³C, ³⁴S), L-Glu (U-¹³C), L-Glu (¹⁵N), L-Glu (U-¹³C, ¹⁵N), L-Glu (U-¹⁸O), L-Glu (U-¹³C, U-¹⁸O), L-Gln (U-¹³C), L-Gln (U-¹⁵N), L-Gln (U-¹³C, U-¹⁵N), L-Gln (U-¹⁸O), L-Gln (U-¹³C, U-¹⁸O), Gly (U-¹³C), Gly (¹⁵N), Gly (U-¹³C, ¹⁵N), Gly (U-¹⁸O), Gly (U-¹³C, U-¹⁸O), L-His (U-¹³C), L-His (U-¹⁵N), L-His (U-¹³C, U-¹⁵N), L-His (U-¹⁸O), L-His (U-¹³C, U-¹⁸O), L-Ile (U-¹³C), L-Ile (¹⁵N), L-Ile (U-¹³C, ¹⁵N), L-Ile (U-¹⁸O), L-Ile (U-¹³C, U-¹⁸O), L-Leu (U-¹³C), L-Leu (¹⁵N), L-Leu (U-¹³C, ¹⁵N), L-Leu (U-¹⁸O), L-Leu (U-¹³C, U-¹⁸O), L-Lys (U-¹³C), L-Lys (U-¹⁵N), L-Lys (U-¹³C, U-¹⁵N), L-Lys (U-¹⁸O), L-Lys (U-¹³C, U-¹⁸O), L-Met (U-¹³C), L-Met (¹⁵N), L-Met (U-¹³C, ¹⁵N), L-Met (U-¹⁸O), L-Met (U-¹³C, U-¹⁸O), L-Met (³⁴S), L-Met (U-¹³C, ³⁴S), L-Phe (U-¹³C), L-Phe (¹⁵N), L-Phe (U-¹³C, ¹⁵N), L-Phe (U-¹⁸O), L-Phe (U-¹³C, U-¹⁸O), L-Pro (U-¹³C), L-Pro (¹⁵N), L-Pro (U-¹³C, ¹⁵N), L-Pro (U-¹⁸O), L-Pro (U-¹³C, U-¹⁸O), L-Ser (U-¹³C), L-Ser (¹⁵N), L-Ser (U-¹³C, ¹⁵N), L-Ser (U-¹⁸O), L-Ser (U-¹³C, U-¹⁸O), L-Thr (U-¹³C), L-Thr (¹⁵N), L-Thr (U-¹³C, ¹⁵N), L-Thr (U-¹⁸O), L-Thr (U-¹³C, U-¹⁸O), L-Trp (U-¹³C), L-Trp (U-¹⁵N), L- Trp (U-¹³C, U-¹⁵N), L-Trp (U-¹⁸O), L-Trp (U-¹³C, U-¹⁸O), L-Tyr (U-¹³C), L-Tyr (¹⁵N), L-Tyr (U-¹³C, ¹⁵N), L-Tyr (U-¹⁸O), L-Tyr (U-¹³C, U-¹⁸O), L-Val (U-¹³C), L-Val (¹⁵N), L-Val (U-¹³C, ¹⁵N), L-Val (U-¹⁸O), or L-Val (U-¹³C, U-¹⁸O). It will be understood that any of the stable isotope labeled amino acid(s) mentioned above can also be in the form of a salt of the corresponding amino acid(s). It is particularly preferred that a mixture of two or more (particularly three or more, more preferably five or more, even more preferably eight or more, and still more preferably twelve or more) stable isotope labeled amino acids selected from any of the above-mentioned preferred groups of stable isotope labeled amino acids or from any of the above-mentioned exemplary stable isotope labeled amino acids are prepared by the method of the invention. Accordingly, it is particularly preferred that a mixture of twelve or more stable isotope labeled amino acids are to be prepared by the method provided herein, wherein said stable isotope labeled amino acids are selected from Ala, Arg, Asn, Asp, Cys, Glu, Gln, Gly, His, ile, Leu, Lys, Met, Phe, Pro, Ser, Thr, Trp, Tyr, and Val (all of which are stable isotope labeled, and all of which have the L-configuration), more preferably from Ala, Arg, Asp, Cys, Glu, Gly, His, ile, Leu, Lys, Met, Phe, Pro, Ser, Thr, Trp, Tyr, and Val (all of which are stable isotope labeled, and all of which have the L-configuration), and even more preferably from Ala, Arg, Asp, Glu, Gly, His, ile, Leu, Lys, Phe, Pro, Ser, Thr, Tyr, and Val (all of which are uniformly stable isotope labeled (e.g., uniformly ¹³C-labeled), and all of which have the L-configuration).

The stable isotope labeled monosaccharide(s) to be prepared by the method of the present invention, including in particular the method according to the first, the third or the fourth embodiment, is/are preferably selected from mannose, glucose, galactose, ribose, arabinose, xylose, rhamnose and N-acetylglucosamine, all of which are stable isotope labeled (preferably uniformly stable isotope labeled), and all of which preferably have the D-configuration. More preferably, the stable isotope labeled monosaccharide(s) is/are selected from D-mannose, D-glucose and N-acetyl-D-glucosamine, all of which are uniformly stable isotope labeled (e.g., uniformly ¹³C-labeled). Examples of stable isotope labeled monosaccharide(s) to be prepared by the method of the present invention include, in particular, D-mannose (U-¹³C), D-mannose (U-¹⁸O), D-mannose (U-¹³C, U-¹⁸O), D-glucose (U-¹³C), D-glucose (U-¹⁸O), D-glucose (U-¹³C, U-¹⁸O), D-galactose (U-¹³C), D-galactose (U-¹⁸O), D-galactose (U-¹³C, U-¹⁸O), D-ribose (U-¹³C), D-ribose (U-¹⁸O), D-ribose (U-¹³C, U-¹⁸O), D-arabinose (U-¹³C), D-arabinose (U-¹⁸O), D-arabinose (U-¹³C, U-¹⁸O), D-xylose (U-¹³C), D-xylose (U-¹⁸O), D-xylose (U-¹³C, U-¹⁸O), D-rhamnose (U-¹³C), D-rhamnose (U-¹⁸O), D-rhamnose (U-¹³C, U-¹⁸O), N-acetyl-D-glucosamine (U-¹³C), N-acetyl-D-glucosamine (U-¹⁸O), N-acetyl-D-glucosamine (U-¹³C, U-¹⁸O), N-acetyl-D-glucosamine (¹⁵N), N-acetyl-D-glucosamine (U-¹³C, ¹⁵N), N-acetyl-D-gtucosamine (U-¹⁸O, ¹⁵N), or N-acetyl-D-glucosamine (U-¹³C, U-¹⁸O,¹⁵N).

The stable isotope labeled lipid(s) to be prepared by the method of the present invention, including in particular the method according to the fifth embodiment, is/are preferably selected from fatty acids, glycerolipids, glycerophospholipids, sphingolipids, sterol lipids, prenol lipids, saccharolipids, and polyketides, all of which are stable isotope labeled (and all of which preferably are uniformly stable isotope labeled). More preferably, the stable isotope labeled lipid(s) is/are selected from fatty acids, glycerolipids, glycerophospholipids, sphingolipids, and sterol lipids, all of which are stable isotope labeled (and ail of which preferably are uniformly stable isotope labeled). Even more preferably, the stable isotope labeled lipid(s) is/are selected from: fatty acids (FA), particularly a saturated fatty acid or an unsaturated fatty acid; glycerolipids (GL), particularly a diacylglycerol (DAG) or a triacylglycerol (TAG); glycerophospholipids (GP), particularly a phosphatidyl acid (PA), a phosphatidylethanolamine (PE), a phosphatidylcholine (PC), a phosphatidylglycerol (PG), a phosphatidylinositol (PI), a phosphatidylserine (PS), a lysophospholipid (LysoGPL), a cardiolipin (CL), or a dimyristoylphosphoethanolamine (DMPE); sphingolipids (SL), particularly a ceramide (Cer), a hexosyl ceramide (HexCer), an inositolphosphorylceramide (IPC), or a mannosylinositolphosphorylceramide (MIPC); and sterol lipids (ST), particularly an ergosterol; wherein all of the aforementioned lipids are stable isotope labeled (preferably they are all uniformly stable isotope labeled, more preferably uniformly ¹³C-labeled). Examples of stable isotope labeled lipid(s) to be prepared by the method of the present invention include, in particular, FA [18:0], FA [18:1], FA [18:2], FA [18:3], FA [18:4], FA [16:0], FA [16:1], FA [16:2], PA [32:2], PA [32:1], PA [33:3], PA [33:2], PA [33:1], PA [34:4], PA [34:3], PA [34:2], PA [34:1], PA [35:4], PA [35:3], PA [35:2], PA [35:1], PA [36:6], PA [36:5], PA [36:4], PA [36:3], PA [36:2], PA [36:1], PC [32:3], PC [32:2], PC [32:1], PC [33:4], PC [33:3], PC [33:2], PC [33:1], PC [34:6], PC [34:5], PC [34:4], PC [34:3], PC [34:2], PC [34:1], PC [35:6], PC [35:5], PC [35:4], PC [35:3], PC [35:2], PC [35:1], PC [36:7], PC [36:5], PC [36:4], PC [36:3], PC [36:2], PC [36:1], PC [37:4], PC [37:3], PC [37:2], PC [38:4], PC [38:3], PC [38:2], PC [38:1], PE [32:3], PE [32:2], PE [32:1], PE [33:2], PE [33:1], PE [34:5], PE [34:4], PE [34:3], PE [34:2], PE [34:1], PE [35:6], PE [35:5], PE [35:4], PE [35:3], PE [35:2], PE [35:1], PE [36:6], PE [36:5], PE [36:4], PE [36:3], PE [36:2], PE [36:1], PG [32:1], PG [33:1], PG [34:4], PG [34:3], PG [34:2], PG [34:1], PG [36:6], PG [36:5], PG [36:4], PG [36:3], PG [36:2], PG [36:1], PI [32:2], PI [32:1], PI [33:2], PI [33:1], PI [34:3], PI [34:2], PI [34:1], PI [35:2], PI [35:1], PI [36:6], PI [36:5], PI [36:4], PI [36:3], PI [36:2], PI [36:1], PS [32:2], PS [32:1], PS [34:3], PS [34:2], PS [34:1], PS [36:5], PS [36:4], PS [36:3], PS [36:2], PS [36:1], LPA [16:0], LPA [18:2], LPA [18:1], LPE [15:0], LPE [16:1], LPE [16:0], LPE [17:3], LPE [17:2], LPE [17:1], LPE [18:1], LPE [18:0], LPG [16:0], LPG [18:3], LPG [18:2], LPG [18:1], LPC [16:2], LPC [16:1], LPC [16:0], LPC [18:0], IPC [34:0,2], IPC [36:0,2], IPC [36:0,4], IPC [42:0,4], IPC [42:0,5], IPC [44:0,4], IPC [44:0,5], MIPC [42:0,4], MIPC [42:0,5], MIPC [44:0,4], MIPC [44:0,5], CL [70:6], CL [71:9], CL [71:8], CL [72:11], CL [72:10], CL [72:9], CL [72:8], CL [72:7], CL [72:6], CL [72:5], DMPE [36:4], DMPE [36:3], DMPE [36:2], DMPE [36:1], DMPE [38:6], DMPE [38:5], DMPE [38:4], DAG [32:2], DAG [32:1], DAG [33:2], DAG [34:4], DAG [34:3], DAG [34:2], DAG [34:1], DAG [35:6], DAG [35:5], DAG [35:4], DAG [35:3], DAG [35:2], DAG [35:1], DAG [36:7], DAG [36:6], DAG [36:5], DAG [36:4], DAG [36:3], DAG [36:2], DAG [36:1], DAG [38:7], DAG [38:6], DAG [38:5], DAG [38:4], DAG [38:3], DAG [38:2], TAG [50:6], TAG [50:5], TAG [50:4], TAG [50:3], TAG [50:2], TAG [51:6], TAG [51:5], TAG [51:4], TAG [51:3], TAG [51:2], TAG [52:6], TAG [52:5], TAG [52:4], TAG [52:3], TAG [52:2], TAG [53:4], TAG [53:3], TAG [54:7], TAG [54:6], TAG [54:5], TAG [54:4], TAG [54:3], TAG [54:2], TAG [56:5], TAG [56:4], TAG [56:3], Erg[16:0], Erg[16:1], Erg[16:2], Erg[17:1], Erg[18:0], Erg[18:1], Erg[18:2], Erg[18:3], Erg[19:2], Erg[19:3], Erg[20:0], Cer [36:2], Cer [36:1], Cer [36:2] oxidized, Cer [36:1] oxidized, HexCer [35:2], or a salt of any of the aforementioned compounds, wherein all of the aforementioned compounds are uniformly stable isotope labeled (preferably, they are uniformly ¹³C-labeled, or uniformly ¹⁸O-labeled, or uniformly ¹³C/¹⁸O-labeled; most preferably, they are all uniformly ¹³C-labeled).

In the method of the present invention, the stable isotope labeled metabolites may also be provided in the form of a salt of the corresponding compound, which may be a salt formed in the course of the cultivation of the microorganism or in any of the subsequent method steps. In particular, such a salt may be formed, e.g., by protonation of an atom carrying an electron lone pair which is susceptible to protonation, such as an amino group, with an inorganic or organic acid, or may be formed as a salt of an acid group (such as a carboxylic acid group) with a cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts.

The method of preparing one or more stable isotope labeled metabolites according to the present invention, including the method according to the first, second, third, fourth or fifth embodiment described herein above, may comprise, after the step of isolating the one or more stable isotope labeled metabolites from the isolated cell wall and/or the isolated cell membrane, a further step of purifying the one or more stable isotope labeled metabolites. Suitable methods for the purification of amino acids, monosaccharides and/or lipids are known in the art.

The method of preparing one or more stable isotope labeled metabolites may also comprise, after the step of isolating the one or more stable isotope labeled metabolites from the isolated cell wall and/or the isolated cell membrane, a further step of storing the one or more stable isotope labeled metabolites in the form of an aqueous composition in a container/receptacle or in dried form in a container/receptacle, wherein the container/receptacle may be placed in a packaging.

Accordingly, the method may comprise, after the step of isolating the one or more stable isotope labeled metabolites from the isolated cell wall and/or the isolated cell membrane, a further step of providing the one or more stable isotope labeled metabolites in the form of an aqueous composition or in dried form, placing the one or more stable isotope labeled metabolites in a container/receptacle, and optionally placing the container/receptacle in a packaging.

The one or more stable isotope labeled metabolites can be provided in dried form by drying the composition containing the one or more stable isotope labeled metabolites using, e.g., spray drying, freeze-drying or any other suitable drying technique. A corresponding dried preparation (e.g., a dried clot or a dried powder) may be reconstituted in a suitable liquid (e.g., in an aqueous solution) prior to its use, e.g., as an internal standard for mass spectrometry or NMR.

The one or more stable isotope labeled metabolites can also be provided in the form of an aqueous composition, e.g., by dialysis or by drying and subsequent reconstitution in the aqueous composition. The aqueous composition, in which the one or more stable isotope labeled metabolites may be stored, preferably comprises about 0.02 M to about 0.5 M hydrochloric acid, more preferably about 0.1 M hydrochloric acid.

The present invention also relates to a composition comprising a mixture of stable isotope labeled metabolites selected from stable isotope labeled amino acids, stable isotope labeled monosaccharides and/or stable isotope labeled lipids, which is obtainable by the method of preparing one or more stable isotope labeled metabolites according to the invention, including the method according to the first, second, third, fourth or fifth embodiment described herein above.

The stable isotope labeled metabolites comprised in said composition may be selected from stable isotope labeled amino acids and/or stable isotope labeled monosaccharides. Accordingly, the mixture of stable isotope labeled metabolites may comprise (or consist of) two or more stable isotope labeled amino acids, preferably three or more stable isotope labeled amino acids, more preferably five or more stable isotope labeled amino acids, even more preferably eight or more stable isotope labeled amino acids, and still more preferably twelve or more stable isotope labeled amino acids. The mixture of stable isotope labeled metabolites may also comprise (or consist of) two or more stable isotope labeled monosaccharides, preferably three or more stable isotope labeled monosaccharides, more preferably four or more stable isotope labeled monosaccharides; or the mixture may comprise (or consist of) one or more stable isotope labeled amino acids and one or more stable isotope labeled monosaccharides, preferably two or more stable isotope labeled amino acids and one or more stable isotope labeled monosaccharides, more preferably three or more stable isotope labeled amino acids and two or more stable isotope labeled monosaccharides, even more preferably five or more stable isotope labeled amino acids and two or more stable isotope labeled monosaccharides, yet even more preferably eight or more stable isotope labeled amino acids and two or more stable isotope labeled monosaccharides, and still more preferably twelve or more stable isotope labeled amino acids and three or more stable isotope labeled monosaccharides. The stable isotope labeled amino acids and/or the stable isotope labeled monosaccharides may be selected from any of the exemplary or preferred stable isotope labeled amino acids and/or from any of the exemplary or preferred stable isotope labeled monosaccharides that have been described herein above in connection with the method of the present invention. Corresponding compositions can be obtained, in particular, by the method according to the first, second, third or fourth embodiment described above.

Alternatively, the stable isotope labeled metabolites comprised in said composition may be selected from stable isotope labeled lipids. Accordingly, the mixture of stable isotope labeled metabolites may comprise (or consist of) two or more stable isotope labeled lipids, preferably three or more stable isotope labeled lipids, more preferably five or more stable isotope labeled lipids, even more preferably ten or more stable isotope labeled lipids, yet even more preferably 20 or more stable isotope labeled lipids, yet even more preferably 30 or more stable isotope labeled lipids, yet even more preferably 40 or more stable isotope labeled lipids, yet even more preferably 50 or more stable isotope labeled lipids, yet even more preferably 75 or more stable isotope labeled lipids, and still more preferably 100 or more stable isotope labeled lipids. These stable isotope labeled lipids may be selected from any of the exemplary or preferred stable isotope labeled lipids described herein above in connection with the method of the present invention. Corresponding compositions can be obtained, in particular, by the method according to the fifth embodiment described above.

Moreover, the stable isotope labeled metabolites prepared in accordance with the present invention can also be combined to provide a mixture of one or more stable isotope labeled amino acids and/or one or more stable isotope labeled monosaccharides with one or more stable isotope labeled lipids. Accordingly, the mixture of stable isotope labeled metabolites may comprise (or consist of) one or more stable isotope labeled amino acids and one or more stable isotope labeled lipids, preferably three or more stable isotope labeled amino acids and three or more stable isotope labeled lipids, more preferably five or more stable isotope labeled amino acids and five or more stable isotope labeled lipids, even more preferably eight or more stable isotope labeled amino acids and ten or more stable isotope labeled lipids, yet even more preferably twelve or more stable isotope labeled amino acids and 20 or more stable isotope labeled lipids. The mixture of stable isotope labeled metabolites may also comprise (or consist of) one or more stable isotope labeled amino acids, one or more stable isotope labeled monosaccharides and one or more stable isotope labeled lipids, preferably three or more stable isotope labeled amino acids, two or more stable isotope labeled monosaccharides and three or more stable isotope labeled lipids, more preferably five or more stable isotope labeled amino acids, two or more stable isotope labeled monosaccharides and five or more stable isotope labeled lipids, even more preferably eight or more stable isotope labeled amino acids, two or more stable isotope labeled monosaccharides and ten or more stable isotope labeled lipids, yet even more preferably twelve or more stable isotope labeled amino acids, three or more stable isotope labeled monosaccharides and 20 or more stable isotope labeled lipids. The stable isotope labeled amino acids, stable isotope labeled monosaccharides and stable isotope labeled lipids may be selected from any of the exemplary or preferred stable isotope labeled amino acids, stable isotope labeled monosaccharides and stable isotope labeled lipids described herein above in connection with the method of the present invention. Compositions comprising such mixtures can be obtained, in particular, by combining the corresponding stable isotope labeled metabolite(s) prepared using the method according to the invention.

As explained above, the composition according to the present invention comprises a mixture of stable isotope labeled metabolites selected from stable isotope labeled amino acids, stable isotope labeled monosaccharides and/or stable isotope labeled lipids. It is preferred that the mixture of stable isotope labeled metabolites makes up, on a dry weight basis, at least about 60% (w/w) of said composition, more preferably at least about 70% (w/w) of said composition, even more preferably at least about 80% (w/w) of said composition, yet even more preferably at least about 90% (w/w) of said composition, and still more preferably at least about 95% (w/w) of said composition.

The composition comprising a mixture of stable isotope labeled metabolites selected from stable isotope labeled amino acids, stable isotope labeled monosaccharides and/or stable isotope labeled lipids according to the present invention is obtainable (or obtained) by the method of preparing one or more stable isotope labeled metabolites provided herein. The composition may therefore contain further components from the cell wall and/or the cell membrane of the microorganism, from which the stable isotope labeled metabolites were isolated. In particular, the composition may comprise one or more polysaccharides from the cell wall and/or from the cell membrane of the microorganism. Such polysaccharides may be selected from mannans, β-glucans, chitin, galactomannans, glucomannans, rhamnomannans, and phosphomannans. Typically, the composition contains at least one polysaccharide (or other component) that originates from and is characteristic of the cell wall and/or the cell membrane of the microorganism.

The present invention furthermore provides various uses of the composition comprising a mixture of stable isotope labeled metabolites selected from stable isotope labeled amino acids, stable isotope labeled monosaccharides and/or stable isotope labeled lipids, which is obtainable by the method of preparing one or more stable isotope labeled metabolites according to the invention.

In particular, the present invention relates to the use of this composition as an internal standard for mass spectrometry or as an internal standard for NMR spectroscopy. The use of stable isotope labeled compounds as internal standards is well-known in the art (see, e.g., Birkemeyer C et al., Trends Biotechnol, 2005, 23(1):28-33; Wu L et al., Anal Biochem, 2005, 336(2):164-71; or Stokvis E et al., Rapid Commun Mass Spectrom, 2005, 19(3):401-7). In particular, a stable isotope labeled compound can be added in a defined (known) concentration to a sample to be analyzed in order to facilitate the qualitative and/or quantitative detection of a corresponding (unlabeled) compound/analyte in the sample by mass spectrometry or NMR spectroscopy; detecting the signals of both the internal standard and the corresponding analyte in the sample and correlating them to a calibration curve established with the same concentration of the internal standard and different (known) concentrations of the corresponding analyte allows to quantitate the amount of analyte contained in the sample.

The invention also relates to the use of this composition as an isotopic tracer, particularly as a metabolic tracer. This allows to track the passage of the stable isotope labeled metabolite(s) through a chemical reaction, a metabolic pathway, or the like, which is useful in order to elucidate chemical reaction mechanisms or metabolic pathways, e.g., of pharmaceuticals, toxins, or nutrients that are administered to an organism (such as a human, a non-human animal, a plant, a fungus, or a bacterium) or an organ or a cell thereof (see, e.g., Birkemeyer C et al., Trends Biotechnol, 2005, 23(1):28-33; Wu L et al., Anal Biochem, 2005, 336(2):164-71; or Buchholz A et al., Biomol Eng, 2002, 19(1):5-15). Accordingly, the present invention also encompasses the use of the above-mentioned composition as a tracer in metabolic flux analysis. The composition can further be used as a dietary tracer, e.g., to study the absorption and/or the metabolism of stable isotope labeled amino acids, stable isotope labeled monosaccharides and/or stable isotope labeled lipids, or the effects of various diseases, disorders and conditions (including, e.g., metabolic disorders, diabetes, obesity, malnutrition, or aging) on the absorption or metabolism of the corresponding stable isotope labeled compounds.

The present invention furthermore relates to the use of this composition as an ingredient for a culture medium. Accordingly, the composition can be used to prepare a culture medium that contains the stable isotope labeled amino acid(s), the stable isotope labeled monosaccharide(s) and/or the stable isotope labeled lipid(s) originally comprised in the composition. This allows the preparation of complex media (or other culture media) which can be used, e.g., for culturing and isotopically labeling bacteria, fungi (e.g., yeasts), animal cells or plant cells. Such culture media can also be used to cultivate and isotopically label cancer cells.

The following definitions apply throughout the present specification, unless specifically indicated otherwise.

As used herein, the term "stable isotope" refers to an isotope that is not radioactive, i.e. that does not undergo radioactive decay, or an isotope that is radioactive but has an extremely long half life of at least 10²⁰ years. A "stable isotope" to be used as a label in accordance with the present invention refers to a stable isotope other than the most abundant naturally occurring isotope of the corresponding element, i.e., to a stable isotope having a different number of neutrons than the most abundant naturally occurring isotope of the corresponding element. For instance, examples of stable isotopes of carbon, nitrogen, oxygen and sulfur to be used as labels in accordance with the present invention include ¹³C, ¹⁵N, ¹⁷O, ¹⁸O, ³³S and ³⁴S but not the most abundant naturally occurring stable isotopes ¹²C, ¹⁴N, ¹⁶O and ³²S. The term "stable isotope" preferably refers to a heavy stable isotope, i.e. a stable isotope having a greater number of neutrons (or a greater atomic mass) than the naturally occurring most abundant stable isotope of the corresponding element. The atomic masses and the natural abundances of different isotopes are known in the art and are described, e.g., in: Meija J et al., Pure and Applied Chemistry, 2016, 88(3):265-91; or Meija J et al., Pure and Applied Chemistry, 2016, 88(3):293-306.

The term "uniformly labeled", as used herein when referring, e.g., to a compound that is "uniformly labeled with a stable isotope" or that is "uniformly stable isotope labeled", indicates that the respective stable isotope replaces all atoms of the corresponding element comprised in the compound. Thus, for example, the term "uniformly ¹³C-labeled" indicates that all carbon atoms comprised in the corresponding compound are replaced by ¹³C.

As used herein, the term "isotope enrichment" (or "isotopic enrichment") or "stable isotope enrichment" (or "stable isotopic enrichment") refers to the percentage of atoms at each of one or more specific atom sites in a compound that are replaced by the corresponding isotope. This term thus indicates the percentage of molecules having a particular (heavy) isotope at one specific atom site, or the percentages of molecules having a particular (heavy) isotope at each of several specific atom sites. For example, if only one atom site is labeled/enriched with a stable isotope (e.g., if only one specific carbon atom out of the six carbon atoms of glucose is replaced with ¹³C, as in glucose-1-¹³C), then the "stable isotope enrichment" indicates what percentage of this atom site is labeled with the corresponding stable isotope. If two (or more) atom sites are labeled/enriched with a stable isotope (e.g., if two specific carbon atoms out of the six carbon atoms of glucose are each replaced with ¹³C, as in glucose-1,2-¹³C₂), then the "stable isotope enrichment" indicates what percentage of each one of these atom sites is labeled with the corresponding stable isotope; for instance, glucose-1,2-¹³C₂ having a "¹³C-enrichment" of 99 atom-% means that 99% of the glucose molecules have ¹³C in position C1 (regardless of whether or not they have ¹³C in position C2) and 99% of the glucose molecules have ¹³C in position C2 (regardless of whether or not they have ¹³C in position C1), whereas the percentage of glucose molecules having ¹³C in both positions C1 and C2 may be lower (typically, it will be (99%)² = 98.01 %). The "isotope enrichment" or "stable isotope enrichment", also when used in connection with uniformly labeled compounds, thus refers to the enrichment in respect of each one of the labeled/enriched positions/atom sites. In accordance with the above explanations, the term "isotope enrichment" or "stable isotope enrichment" thus refers to the percentage of a particular isotope which would be assessed if the corresponding molecule or compound was 100% atomized; this isotopic abundance then translates into an isotopologue pattern (multi-nominal distributions) for the molecule/compound; in other words, in the exemplary case of a compound having a ¹³C-enrichment of 99.9%, the bigger the compound the higher the probability that a ¹²C isotopologue is present (despite the 99.9% abundance of ¹³C in the compound). The stable isotope enrichment of a given compound can be determined using methods known in the art, particularly by mass spectrometry.

The term "metabolite", as used herein in connection with, e.g., the preparation of one or more stable isotope labeled metabolites in a microorganism, refers to any substance that is produced by (or within) the corresponding microorganism, or any component or fragment of such a substance.

The terms "peptide" and "protein", as in the expressions "peptides and/or proteins" and "peptides/proteins", are used herein interchangeably and refer to a polymer of two or more amino acids linked via amide bonds that are formed between an amino group of one amino acid and a carboxyl group of another amino acid. The amino acids comprised in the peptide or protein, which are also referred to as amino acid residues, may be selected from the 20 standard proteinogenic α-amino acids (i.e., Ala, Arg, Asn, Asp, Cys, Glu, Gin, Gly, His, ile, Leu, Lys, Met, Phe, Pro, Ser, Thr, Trp, Tyr, and Val) but also from non-proteinogenic and/or non-standard α-amino acids (such as, e.g., ornithine, citrulline, homolysine, pyrrolysine, 4-hydroxyproline, α-methylalanine (i.e., 2-aminoisobutyric acid), norvaline, norleucine, terleucine (tert-leucine), labionin, or an alanine or glycine that is substituted at the side chain with a cyclyl group (e.g., a cycloalkyl group, a heterocycloalkyl group, an aryl group, or a heteroaryl group) like, e.g., cyclopentylalanine, cyclohexylalanine, phenylalanine, naphthylalanine, pyridylalanine, thienylalanine, cyclohexylglycine, or phenylglycine) as well as β-amino acids (e.g., β-alanine), γ-amino acids (e.g., γ-aminobutyric acid, isoglutamine, or statine) and δ-amino acids. Preferably, the amino acid residues comprised in the peptide or protein are selected from α-amino acids, more preferably from the 20 standard proteinogenic α-amino acids (which can be present as the L-isomer or the D-isomer, and are preferably all present as the L-isomer). The peptide or protein may be unmodified or may be modified, e.g., at its N-terminus, at its C-terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the glycosylation (i.e., the covalent attachment of one or more sugar moieties) and/or the acylation with one or more fatty acids (e.g., one or more C₈₋₃₀ alkanoic or alkenoic acids; forming a fatty acid acylated peptide or protein). Accordingly, the "peptide" or "protein" may also be a glycopeptide or a glycoprotein. The amino acid residues comprised in the peptide or protein may, e.g., be present as a linear molecular chain (forming a linear peptide or protein) or may form one or more rings (corresponding to a cyclic peptide or protein). The peptide or protein may also form oligomers consisting of two or more identical or different molecules.

As used herein, the term "amino acid" refers to a compound comprising at least one carboxylic acid group and at least one amino group. An "amino acid" may be an α-amino acid, particulary any one of the 20 standard proteinogenic α-amino acids (i.e., Ala, Arg, Asn, Asp, Cys, Glu, Gln, Gly, His, ile, Leu, Lys, Met, Phe, Pro, Ser, Thr, Trp, Tyr, or Val) but also a non-proteinogenic and/or non-standard α-amino acid (such as, e.g., ornithine, citrulline, homolysine, pyrrolysine, 4-hydroxyproline, α-methylalanine (i.e., 2-aminoisobutyric acid), norvaline, norleucine, terleucine (tert-leucine), labionin, or an alanine or glycine that is substituted at the side chain with a cyclic group (e.g., a cycloalkyl group, a heterocycloalkyl group, an aryl group, or a heteroaryl group) like, e.g., cyclopentylalanine, cyclohexylalanine, phenylalanine, naphthylalanine, pyridylalanine, thienylalanine, cyclohexylglycine, or phenylglycine), or a β-amino acid (e.g., β-alanine), a γ-amino acid (e.g., γ-aminobutyric acid, isoglutamine, or statine), or a δ-amino acid. Unless defined otherwise, the term "amino acid" preferably refers to an α-amino acid, more preferably to any one of the 20 standard proteinogenic α-amino acids (which may be in the form of the L-isomer or the D-isomer but are preferably in the form of the L-isomer).

As used herein, the term "polysaccharide" refers to a linear or branched polymer of several (e.g., 10 or more, particularly 40 or more) monosaccharides, which may be the same or different monosaccharides and which are linked via glycosidic bonds, or to a compound containing at least one such polymer as a subunit/moiety. Accordingly, a "polysaccharide" may be a polymer composed solely of monosaccharides, or it may additionally comprise other non-sugar subunits/moieties.

As used herein, the term "monosaccharide" refers to a sugar/carbohydrate that cannot be hydrolyzed to a simpler sugar/carbohydrate, and also includes sugar derivatives such as amino sugars (like, e.g., glucosamine or N-acetylglucosamine). Monosaccharides can be aldoses or ketoses, and can also be deoxy sugars. Examples of a "monosaccharide" include, in particular, a triose (e.g., glyceraldehyde or dihydroxyacetone), a tetrose (e.g., erythrose, threose, or erythrulose), a pentose (e.g., arabinose, lyxose, ribose, deoxyribose, xylose, ribulose, or xylulose), a hexose (e.g., allose, altrose, galactose, glucose, gulose, idose, mannose, talose, fructose, psicose, sorbose, tagatose, fucose, fuculose, rhamnose, galactosamine, glucosamine, or N-acetylglucosamine), or a heptose (e.g., mannoheptulose or sedoheptulose).

As used herein, the term "lipid" refers to a hydrophobic or amphiphilic compound, and in particular refers to a fatty acid, a glycerolipid, a glycerophospholipid, a sphingolipid, a sterol lipid, a prenol lipid, a saccharolipid, or a polyketide (see, e.g., Fahy E et al., J Lipid Res, 2005, 46(5):839-61; Fahy E et al., J Lipid Res, 2009, 50 Suppl:S9-14; or Fahy E et al., Biochim Biophys Acta, 2011, 1811(11):637-47).

As used herein, the term "alkyl" refers to a monovalent saturated acyclic (i.e., non-cyclic) hydrocarbon group (i.e., a group consisting of carbon atoms and hydrogen atoms) which may be linear or branched. Accordingly, an "alkyl" group does not comprise any carbon-to-carbon double bond or any carbon-to-carbon triple bond. A "C₁₋₆ alkyl" denotes an alkyl group having 1 to 6 carbon atoms. Exemplary alkyl groups are methyl, ethyl, propyl (e.g., n-propyl or isopropyl), or butyl (e.g., n-butyl, isobutyl, sec-butyl, or tert-butyl).

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₆ alkenyl" denotes an alkenyl group having 2 to 6 carbon atoms. Exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl).

As used herein, the term "alkanoic acid" refers to a compound R-COOH, wherein R is an alkyl group. A "C₈₋₃₀ alkanoic acid" denotes an alkanoic acid having 8 to 30 carbon atoms (such as, e.g., an alkanoic acid having 8, 9, 10, 11, 12, 13, 14, 15, 16, 17, 18, 19, 20, 21, 22, 23 or 24 carbon atoms).

As used herein, the term "alkenoic acid" refers to a compound R-COOH, wherein R is an alkenyl group. A "C₈₋₃₀ alkenoic acid" denotes an alkenoic acid having 8 to 30 carbon atoms (such as, e.g., an alkenoic acid having 8, 9, 10, 11, 12, 13, 14, 15, 16, 17, 18, 19, 20, 21, 22, 23 or 24 carbon atoms).

As used herein, the term "about" preferably refers to ±10% of the indicated numerical value, more preferably to ±5% of the indicated numerical value, and in particular to the exact numerical value indicated. For example, the expression "about 100" preferably refers to 100 ±10%, more preferably to 100 ±5%, and even more preferably to the specific value of 100. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint.

As used herein, the term "comprising" (or "comprise", "comprises", "contain", "contains", "containing", or the like), unless explicitly indicated otherwise or contradicted by context, has the meaning of "containing, inter alia", i.e., "containing, among further optional elements, ...". In addition thereto, this term also specifically includes the narrower meanings of "consisting essentially of" and "consisting of". For example, the term "A comprising B and C" has the meaning of "A containing, inter alia, B and C", wherein A may contain further optional elements/components/steps (e.g., "A containing B, C and D" would also be encompassed), but this term also specifically includes the meaning of "A consisting essentially of B and C" and the meaning of "A consisting of B and C" (i.e., no other components than B and C are comprised in A).

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, if a certain step of a method is indicated to be "optional", the invention specifically relates to both possibilities, i.e., that this step is to be conducted (i.e., forms part of the corresponding method) or that this step is not to be conducted (i.e., does not form part of the corresponding method). Likewise, if a component of a composition is indicated to be "optional", the invention specifically relates to both possibilities, i.e., that the corresponding component is present (contained in the composition) or that the corresponding component is absent from the composition.

As used herein, unless explicitly indicated otherwise or contradicted by context, the terms "a", "an" and "the" are used interchangeably with "one or more" and "at least one". Thus, for example, a composition comprising "a" component X can be interpreted as referring to a composition comprising "one or more" components X.

Unless specifically indicated otherwise, all properties and parameters referred to herein (including, e.g., any amounts/concentrations indicated in "% (v/v)", in "% (w/v)" or in "mg/ml", as well as any pH values) are preferably to be determined at standard ambient temperature and pressure conditions, particularly at a temperature of 25°C (298.15 K) and at an absolute pressure of 101.325 kPa (1 atm).

The different method steps of the methods provided herein can, in general, be carried out in any suitable order, unless indicated otherwise or contradicted by context, and are preferably carried out in the specific order in which they are indicated.

It is to be understood that the present invention specifically relates to each and every combination of features described herein, including any combination of general and/or preferred features. In particular, the invention specifically relates to all combinations of preferred features of the methods, compositions and uses provided herein.

In this specification, a number of documents including patents, patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

The present invention particularly relates to the following items:
1. A method of preparing one or more stable isotope labeled metabolites selected from stable isotope labeled amino acids, stable isotope labeled monosaccharides, stable isotope labeled lipids, and mixtures thereof, the method comprising:
   - cultivating a microorganism in the presence of a stable isotope labeled nutrient;
   - isolating the cell wall and the cell membrane of the microorganism; and
   - isolating said one or more stable isotope labeled metabolites from the isolated cell wall and/or the isolated cell membrane.
2. The method of item 1, wherein said one or more stable isotope labeled metabolites are one or more stable isotope labeled amino acids and/or one or more stable isotope labeled monosaccharides, and
   wherein the step of isolating said one or more stable isotope labeled metabolites from the isolated cell wall and/or the isolated cell membrane is conducted by:
   - extracting peptides/proteins and/or polysaccharides from the isolated cell wall and/or the isolated cell membrane;
   - hydrolyzing said peptides/proteins and/or said polysaccharides and thereby obtaining a mixture of stable isotope labeled amino acids and/or stable isotope labeled monosaccharides; and
   - optionally, isolating one or more stable isotope labeled amino acids and/or one or more stable isotope labeled monosaccharides from said mixture.
3. The method of item 1 or 2, wherein said one or more stable isotope labeled metabolites are one or more stable isotope labeled amino acids, and
   wherein the step of isolating said one or more stable isotope labeled metabolites from the isolated cell wall and/or the isolated cell membrane is conducted by:
   - extracting peptides and/or proteins from the isolated cell wall and/or the isolated cell membrane;
   - hydrolyzing said peptides and/or proteins and thereby obtaining a mixture of stable isotope labeled amino acids; and
   - optionally, isolating one or more stable isotope labeled amino acids from said mixture.
4. The method of item 1 or 2, wherein said one or more stable isotope labeled metabolites are one or more stable isotope labeled monosaccharides, and
   wherein the step of isolating said one or more stable isotope labeled metabolites from the isolated cell wall and/or the isolated cell membrane is conducted by:
   - extracting polysaccharides from the isolated cell wall and/or the isolated cell membrane;
   - hydrolyzing said polysaccharides and thereby obtaining a mixture of stable isotope labeled monosaccharides; and
   - optionally, isolating one or more stable isotope labeled monosaccharides from said mixture.
5. The method of item 1, wherein said one or more stable isotope labeled metabolites are one or more stable isotope labeled lipids, and
   wherein the step of isolating said one or more stable isotope labeled metabolites from the isolated cell wall and/or the isolated cell membrane is conducted by:
   - extracting lipids from the isolated cell wall and/or the isolated cell membrane and thereby obtaining a mixture of stable isotope labeled lipids; and
   - optionally, isolating one or more stable isotope labeled lipids from said mixture.
6. The method of any one of items 1 to 5, wherein the stable isotope is selected from ¹³C, ¹⁵N, ¹⁷O, ¹⁸O, ³³S, ³⁴S and any combination thereof.
7. The method of any one of items 1 to 6, wherein the stable isotope is a single stable isotope selected from ¹³C, ¹⁵N, ¹⁷O, ¹⁸O, ³³S, and ³⁴S.
8. The method of any one of items 1 to 6, wherein the stable isotope is a combination of two or more stable isotopes which are each selected from ¹³C, ¹⁵N, ¹⁷O, ¹⁸O, ³³S , and ³⁴S.
9. The method of any one of items 1 to 8, wherein said stable isotope(s) is/are selected from ¹³C, ¹⁵N, and ³⁴S.
10. The method of any one of items 1 to 6, wherein the stable isotope is ¹³C, optionally in combination with ¹⁵N and/or ³⁴S.
11. The method of any one of items 1 to 10, wherein the one or more stable isotope labeled metabolites are uniformly labeled with said stable isotope.
12. The method of any one of items 1 to 8, wherein the one or more stable isotope labeled metabolites have an isotope enrichment of at least about 95 atom-%.
13. The method of any one of items 1 to 12, wherein the one or more stable isotope labeled metabolites have an isotope enrichment of at least about 98 atom-%.
14. The method of any one of items 1 to 5, wherein the stable isotope is ¹³C, and wherein the one or more stable isotope labeled metabolites are uniformly ¹³C-labeled metabolites having a ¹³C enrichment of at least about 95 atom-%.
15. The method of any one of items 1 to 3 or 6 to 14, wherein the stable isotope labeled amino acid(s) is/are selected from Ala, Arg, Asn, Asp, Cys, Glu, Gln, Gly, His, ile, Leu, Lys, Met, Phe, Pro, Ser, Thr, Trp, Tyr, and Val, all of which are stable isotope labeled.
16. The method of any one of items 1, 2, 4 or 6 to 14, wherein the stable isotope labeled monosaccharide(s) is/are selected from mannose, glucose, galactose, ribose, arabinose, xylose, rhamnose and N-acetylglucosamine, all of which are stable isotope labeled.
17. The method of any one of items 1 or 5 to 14, wherein the stable isotope labeled lipid(s) is/are selected from fatty acids, glycerolipids, glycerophospholipids, sphingolipids, sterol lipids, prenol lipids, saccharolipids, and polyketides, all of which are stable isotope labeled.
18. The method of any one of items 1 to 17, wherein said microorganism is cultivated in a culture medium in a fermenter, wherein the culture medium contains said stable isotope labeled nutrient, and wherein the temperature of the culture medium is about 25°C to about 32°C.
19. The method of any one of items 1 to 18, wherein said microorganism is a fungus or a bacterium.
20. The method of any one of items 1 to 19, wherein said microorganism is a yeast.
21. The method of any one of items 1 to 20, wherein said microorganism is a yeast of the genus *Pichia, Saccharomyces, Candida, Hansenula, Schizosaccharomyces,* or *Kluyveromyces.*
22. The method of any one of items 1 to 21, wherein said microorganism is *Pichia pastoris.*
23. The method of any one of items 1 to 22, wherein said stable isotope labeled nutrient is a stable isotope labeled sugar.
24. The method of any one of items 1 to 7 or 9 to 23, wherein said stable isotope is ¹³C, wherein said stable isotope labeled nutrient is ¹³C-labeled glucose, and wherein said ¹³C-labeled glucose is the sole carbon source in the presence of which the microorganism is cultivated.
25. The method of any one of items 1 to 24, wherein said microorganism is cultivated in a culture medium containing uniformly ¹³C-labeled glucose having a ¹³C enrichment of at least about 98 atom-% as the sole carbon source.
26. The method of any one of items 1 to 25, wherein said stable isotope uniformly labels all nutrients that constitute sources of the corresponding element and in the presence of which the microorganism is cultivated.
27. The method of any one of items 1 to 26, wherein, after the step of cultivating the microorganism and prior to the step of isolating the cell wall and the cell membrane of the microorganism, the method comprises a step of quenching the cells of the microorganism.
28. The method of item 27, wherein the step of quenching the cells of the microorganism is conducted using methanol at a temperature of about -15°C to about -45°C.
29. The method of any one of items 1 to 28, wherein the step of isolating the cell wall and the cell membrane of the microorganism is conducted by: (i) boiling the cells of the microorganism in an ethanol/water mixture, in a methanol/water mixture or in a propanol/water mixture, and then isolating the cell wall and the cell membrane by filtration and/or centrifugation; or (ii) subjecting the cells of the microorganism to a freeze-thaw treatment, and then isolating the cell wall and the cell membrane by filtration and/or centrifugation; or (iii) subjecting the cells of the microorganism to sonication, and then isolating the cell wall and the cell membrane by filtration and/or centrifugation; or (iv) treating the cells of the microorganism with a detergent, and then isolating the cell wall and the cell membrane by filtration and/or centrifugation.
30. The method of any one of items 1 to 29, wherein the step of isolating the cell wall and the cell membrane of the microorganism is conducted by boiling the cells of the microorganism in an ethanol/water mixture and filtering off the cell wall and the cell membrane.
31. The method of any one of items 1 to 30, wherein, after the step of isolating the cell wall and the cell membrane of the microorganism and prior to the step of isolating the one or more stable isotope labeled metabolites from the isolated cell wall and/or the isolated cell membrane, the method comprises a step of subjecting the isolated cell wall and the isolated cell membrane to mechanical disruption.
32. The method of item 2 or 3 or any one of their dependent items 6 to 31, wherein the peptides and/or proteins are extracted from the isolated cell wall and/or the isolated cell membrane using one or more precipitation methods selected from chloroform/methanol precipitation, methyl-tert-butyl ether precipitation, diethyl ether precipitation, acetone precipitation, acidified acetone/methanol precipitation, trichloroacetic acid precipitation, and ammonium sulfate precipitation.
33. The method of item 2 or 4 or any one of their dependent items 6 to 31, wherein the step of extracting polysaccharides from the isolated cell wall and/or the isolated cell membrane is conducted using an organic solvent.
34. The method of item 33, wherein said organic solvent is selected from methyl-tert-butyl ether, chloroform/methanol, dichloromethane/methanol, hexane, hexane/isopropanol, acetone/ethanol, ethanol/diethyl ether, and diethyl ether.
35. The method of item 2 or any one of its dependent items 3, 4 or 6 to 34, wherein the peptides/proteins and/or the polysaccharides are hydrolyzed under acidic conditions.
36. The method of item 5 or any one of its dependent items 6 to 31, wherein the step of extracting lipids from the isolated cell wall and/or the isolated cell membrane is conducted using an organic solvent.
37. The method of item 36, wherein said organic solvent is selected from methyl-tert-butyl ether, chloroform/methanol, dichloromethane/methanol, hexane, hexane/isopropanol, acetone/ethanol, ethanol/diethyl ether, and diethyl ether.
38. The method of item 36 or 37, wherein said organic solvent is methyl-tert-butyl ether.
39. The method of item 36 or 37, wherein said organic solvent is chloroform/methanol.
40. The method of any one of items 36 to 38, wherein the step of extracting lipids from the isolated cell wall and/or the isolated cell membrane is conducted by suspending the isolated cell wall and/or the isolated cell membrane in methanol, adding methyl-tert-butyl ether, inducing phase separation by adding water or an aqueous solution, and collecting the organic phase containing said lipids.
41. The method of any one of items 36, 37 or 39, wherein the step of extracting lipids from the isolated cell wall and/or the isolated cell membrane is conducted by suspending the isolated cell wall and/or the isolated cell membrane in chloroform/methanol, isolating the liquid phase, inducing phase separation by adding water or an aqueous solution, and collecting the organic phase containing said lipids.
42. The method of any one of items 1 to 41, wherein, after the step of isolating said one or more stable isotope labeled metabolites from the isolated cell wall and/or the isolated cell membrane, the method comprises a step of purifying the one or more stable isotope labeled metabolites.
43. The method of any one of items 1 to 42, wherein, after the step of isolating said one or more stable isotope labeled metabolites from the isolated cell wall and/or the isolated cell membrane, the method comprises a step of storing the one or more stable isotope labeled metabolites in the form of an aqueous composition in a container, wherein the container is optionally placed in a packaging.
44. The method of any one of items 1 to 42, wherein, after the step of isolating said one or more stable isotope labeled metabolites from the isolated cell wall and/or the isolated cell membrane, the method comprises a step of storing the one or more stable isotope labeled metabolites in dried form in a container, wherein the container is optionally placed in a packaging.
45. A composition comprising a mixture of stable isotope labeled metabolites selected from stable isotope labeled amino acids, stable isotope labeled monosaccharides and/or stable isotope labeled lipids, which is obtainable by the method of any one of items 1 to 44.
46. The composition of item 45, wherein said stable isotope labeled metabolites are two or more stable isotope labeled amino acids.
47. The composition of item 45, wherein said stable isotope labeled metabolites are two or more stable isotope labeled monosaccharides.
48. The composition of item 45, wherein said stable isotope labeled metabolites are two or more stable isotope labeled lipids.
49. The composition of item 45, wherein said stable isotope labeled metabolites consist of one or more stable isotope labeled amino acids, one or more stable isotope labeled monosaccharides and one or more stable isotope labeled lipids.
50. The composition of any one of items 45 to 49, wherein said mixture of stable isotope labeled metabolites makes up, on a dry weight basis, at least about 70% (w/w) of said composition.
51. The composition of any one of items 45 to 50, wherein said mixture of stable isotope labeled metabolites makes up, on a dry weight basis, at least about 90% (w/w) of said composition.
52. The composition of any one of items 45 to 51, wherein the composition comprises one or more polysaccharides from the cell wall and/or from the cell membrane of the microorganism.
53. The composition of item 52, wherein said polysaccharides are selected from mannans, β-glucans, chitin, galactomannans, glucomannans, rhamnomannans, and phosphomannans.
54. Use of the composition of any one of items 45 to 53 as internal standard for mass spectrometry or NMR spectroscopy.
55. Use of the composition of any one of items 45 to 53 as metabolic tracer.
56. Use of the composition of any one of items 45 to 53 as an ingredient for a culture medium.

The invention is also described by the following illustrative figures. The appended figures show:
Figure 1: Illustration of the method of preparing one or more stable isotope labeled metabolites according to the present invention.

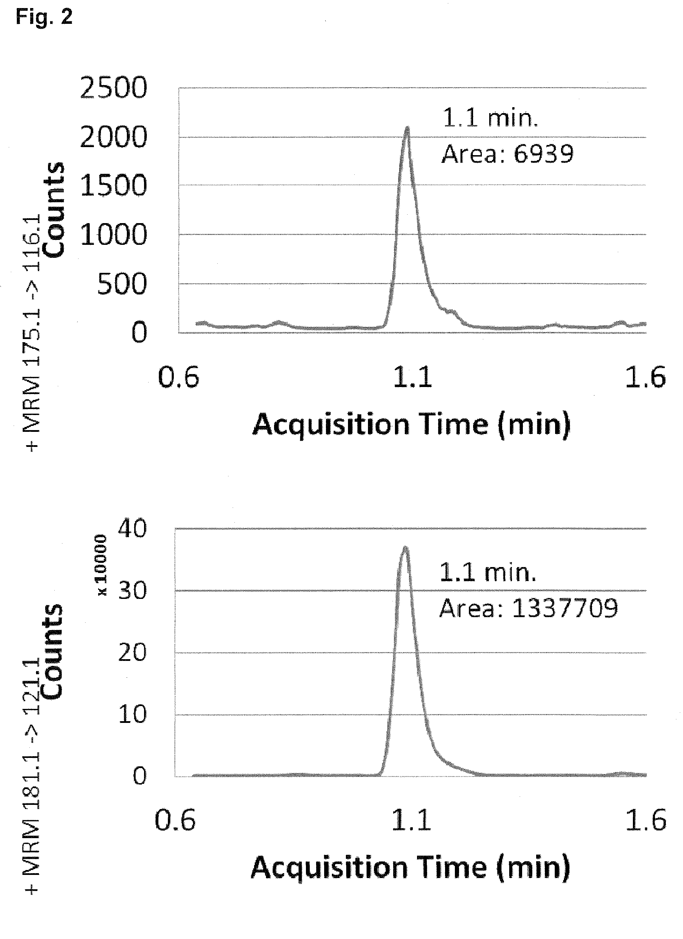

Figure 2: Determination of the isotope enrichment of uniformly ¹³C-labeled arginine comprised in a mixture of uniformly ¹³C-labeled amino acids according to the invention (see Example 1). In this example the monoisotopic peak of the fully ¹³C-labeled arginine (bottom) and the monoisotopic peak of the fully ¹²C (natural) arginine (top) were compared. The area of signal for the ¹³C-labeled molecule was 1337709, the area for the fully ¹²C molecule was 6939. The 6939 are only 0.5% of the total peak area, which indicates that the isotope enrichment in this example is about 99.5%.

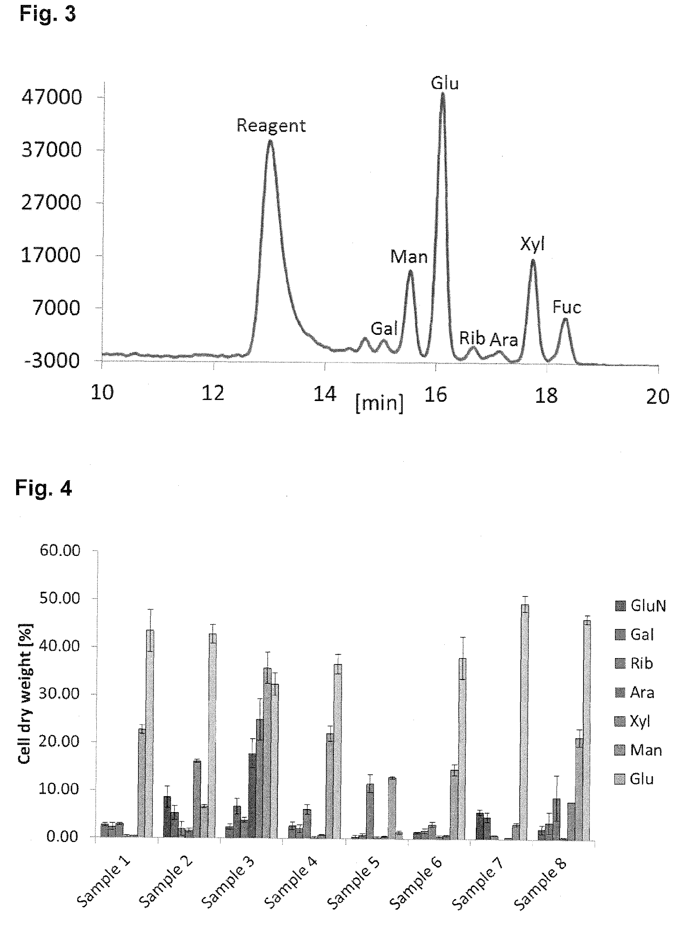

Figure 3: Exemplary chromatogram of the monosaccharides derivatized with anthranilic acid. Eight different yeast cell wall samples (n=3) from *Saccharomyces cerevisiae* were analyzed using the presented method (see Example 3).
Figure 4: Monosaccharide concentrations in % cell dry weight for 8 different *Saccharomyces cerevisiae* samples (n=3 independent replicates). For each sample, the depicted columns (from left to right) refer to the % cell dry weight of glucosamine (GluN), galactose (Gal), ribose (Rib), arabinose (Ara), xylose (Xyl), mannose (Man), and glucose (Glu), respectively. See Example 3.

The invention will now be described by reference to the following examples which are merely illustrative and are not to be construed as a limitation of the scope of the present invention.

### EXAMPLES

### Example 1: Preparation of uniformly ¹³C-labeled amino acids from yeast cell wall

### Reagents

The ¹³C-glucose (∼99% D-glucose ¹³C₆, according to certificate of analysis: 99.4%) was obtained from Sigma Aldrich. The trace salt mix for the fermentation was prepared according to Neubauer S et al., J Sep Sci, 2012, 35(22):3091-105. Ultra pure HCl was obtained by subboiling. Chloroform was purchased from Sigma (Steinheim, Germany). All other reagents were of analytical grade or higher.

### Fermentation of Pichia pastoris for ¹³C-labeled material production

*Pichia pastoris* was fermented on labeled glucose as sole carbon source. Conditions applied were previously described by Neubauer S et al., J Sep Sci, 2012, 35(22):3091-105. Briefly, the cells were grown on ¹³C-glucose (∼99%) to a final cell dry weight of 4.5 g/L to produce labeled yeast. Afterwards, the yeast cells were quenched by methanol followed by boiling ethanol extraction to separate the metabolome from the yeast cell wall. A typical fermentation process led to 2000 mL broth. Ten yeast cell wall pellets (15 mL tubes) with remaining ethanol (∼200 µL solutions) were combined together by adding 1 mL of water to the first tube. After mixing, the solution was poured in the second tube and so on. The tubes were washed by transferring 1 mL water in first tube and use the previous work flow. This procedure was continued with the next 10 tubes until no cell wall containing tubes were left. The remaining solution in the tubes was transferred using a glass pipette. Afterwards, all tubes were combined in a 50 mL falcon tube and extensively mixed prior to subdivision in 5 mL tubes. The cell wall containing solutions were frozen at -80°C for 1 h prior to drying by SpeedVac for approximately 5 h. Finally, the dried material was pulverized using a precleaned glass stick and stored at -20°C until further analysis.

### Yeast cell wall processing (Folch J et al., J Biol Chem, 1957, 226(1):497-509)

Lipid extraction: Aliquots of 20 mg dry ¹³C-labeled yeast cell wall were weighted in glass tubes and 4 mL of a 4:8:3 chloroform:MeOH:H₂O were added to extract the lipids. Everything was placed for 15 min into a sonication bath to dissolve sample powder completely. Afterwards, the samples were shaken for 30 min at room temperature. The samples were put on ice for several minutes to decrease protein solubility in solvent. Then the samples were centrifuged for 10-15 min at 2500-3000 rpm at 4°C. The lipid containing supernatant was removed and the whole procedure was repeated. Finally, another 4 mL of the mixture was added prior to sample mixing, sonication and shaking over night at room temperature.

Protein precipitation: The samples were cooled on ice, and the supernatant was removed after 10-15 min centrifugation at 2500-3000 rpm. 1 mL of cold H₂O (to remove free carbohydrates) and 4 mL cold acetone per sample were added. The samples were placed on ice for 15-30 min prior to 10-15 min of centrifugation at 2500-3000 rpm at 4°C. The supernatant was removed using a glass pipette and the whole procedure was repeated another 3 times. Afterwards, 4 mL cold acetone was added to complete protein precipitation using 10-15 min of centrifugation at 2500-3000 rpm and 4°C. The supernatant was removed carefully to circumvent protein loss from the glass walls. This step was repeated and a final acetone removal step was applied. Then, the samples were dried under N₂ until the samples turned completely white.

### Protein hydrolysis (Gehrke CW et al., Journal of the Association of Official Analytical Chemists, 1985, 68(5):811-21)

Protein hydrolysis was applied with 6 mg of protein. 1 mL of 6 M HCl was added and the samples were hydrolyzed at 100°C for 24 h. The hydrolyzed samples were mixed and centrifuged at 10000 rpm for 5 min and transferred to an Eppendorf tube, evaporated, and either directly used for analysis or stored at -80°C.

### Quantitative analysis of the cell wall extract

The content of amino acids in the hydrolyzed protein extracts obtained from the cell walls was determined via LC-MS/MS. For the measurements a mass spectrometer from Agilent Technologies was used (Model Number 6490 with a 1290 HPLC system). The separation method used was reversed phase chromatography.

For the quantitative determination, an external calibration was established using unlabeled amino acids at 0.001-100 µM and spiked with the hydrolyzed protein extract obtained from the cell walls. The signals of labeled and unlabeled amino acids were placed in relation to determine the concentration of fully ¹³C-labeled amino acids.

For this external calibration, the certified amino acid standard from Fluka (17 amino acids in 0.1 M hydrochloric acid) was used, which contained 2.5 mM of each of alanine, arginine, aspartic acid, glutamic acid, glycine, histidine, isoleucine, leucine, lysine, methionine, phenylalanine, proline, serine, threonine, tyrosine, and valine, and further contained 1.25 mM cystine.

As the production process of the extract obtained from cell walls involves acidic hydrolysis there are some limitations to the product. Glutamine (Gin) is converted to glutamic acid (Glu), asparagine (Asn) is converted to aspartic acid (Asp) and tryptophan is destroyed. Additionally cysteine and methionine are lost in the process. Those 5 amino acids were excluded from the quantification.

Quantification revealed the following concentrations in the measurement solution (hydrolyzed protein extract obtained from cell walls diluted 1:100):

| **Compound** | **Conc. [µM]** | **Relative standard deviation [%]** | **Stddev [µM]** |
|---|---|---|---|
| Lys Results | 2.9 | 7.1 | 0.2 |
| His Results | 2.0 | 3.6 | 0.1 |
| Arg Results | 4.5 | 10 | 0.5 |
| Ser Results | 9.5 | 15 | 1.4 |
| Asp Results | 13 | 15.0 | 2.0 |
| Ala Results | 15 | 5.6 | 0.9 |
| Thr Results | 6.7 | 5.8 | 0.4 |
| Glu Results | 18 | 11 | 1.9 |
| Pro Results | 6.3 | 7.6 | 0.5 |
| Val Results | 4.3 | 4.8 | 0.2 |
| Tyr Results | 1.1 | 3.0 | 0.03 |
| Ile Results | 8.2 | 3.8 | 0.3 |
| Leu Results | 23 | 4.0 | 0.9 |
| Phe Results | 4.0 | 0.6 | 0.02 |
| Gly Results | 9.2 | 2.1 | 0.2 |
| Trp Results | | Destroyed (process) | |
| Cysteine Results | | Destroyed (process) | |
| Cystine Results | | Destroyed (process) | |
| Met Results | | Destroyed (process) | |
| Gln Results | | Transformed to Glu | |
| Asn Results | | Transformed to Asp | |

| | | | |
|---|---|---|---|
| ("Conc.": concentration; "Stddev": standard deviation) | | | |

From these results the following concentrations in the sample solution (which had been diluted 1:100 to obtain the measurement solution) and amounts of obtained amino acids were determined:

| **Compound** | **Molecular weight [g/mol]** | **Conc. [µM] measurement solution** | **Conc. [mM] sample solution** | **Conc. [mg/L] sample solution** | **Amount of amino acids [µg]** * |
|---|---|---|---|---|---|
| C13 Lys | 152 | 2.9 | 0.29 | 44 | 44 |
| C13 His | 161 | 2.0 | 0.2 | 32 | 32 |
| C13 Arg | 180 | 4.5 | 0.45 | 81 | 81 |
| C13 Ser | 108 | 9.5 | 0.95 | 103 | 103 |
| C13 Asp | 137 | 13 | 1.3 | 181 | 181 |
| C13 Ala | 92 | 16 | 1.6 | 146 | 146 |
| C13 Thr | 123 | 6.7 | 0.67 | 82 | 82 |
| C13 Glu | 152 | 18 | 1.8 | 278 | 278 |
| C13 Pro | 120 | 6.3 | 0.63 | 76 | 76 |
| C13 Val | 122 | 4.3 | 0.43 | 53 | 53 |
| C13 Tyr | 190 | 1.1 | 0.11 | 21 | 21 |
| C13 Ile | 137 | 8.2 | 0.82 | 113 | 113 |
| C13 Leu | 137 | 23 | 2.3 | 311 | 311 |
| C13 Phe | 174 | 4 | 0.4 | 70 | 70 |
| C13 Gly | 77 | 9.2 | 0.92 | 71 | 71 |
| | | | | Sum: | 1661 |

| | | | | | |
|---|---|---|---|---|---|
| (* 1 ml sample solution was produced, so in total the indicated amounts of amino acids in µg were obtained) | | | | | |

The sample resulted from the acidic hydrolysis of 6 mg protein powder obtained from the procedure described above. 15 of the 20 proteinogenic amino acids have been quantified. The weight of the 15 amino acids together is 1.6 mg. From this figure it can be estimated that the 20 proteinogenic amino acids amount altogether to approximately 2 mg, which would imply that roughly 30% of the initial 6 mg precipitate (protein powder) are proteins. Those 6 mg were produced of 20 mg of dry yeast cell wall. Calculating back this means that 10% of the yeast cell wall consist of protein. This is in agreement with the literature and gives further proof of the feasibility and the remarkable efficiency of the method according to the present invention.

### Isotope enrichment

The isotope enrichment of the amino acids was also determined (see Figure 2). Some are shown here as examples.

| | |
|---|---|
| Ala | 99.2 |
| Arg | 99.5 |
| Asp | 98.1 |
| Glu | 99.1 |
| His | 99.7 |
| Ile | 99.7 |
| Leu | 99.8 |
| Phe | 99.5 |
| Pro | 99.0 |
| Tyr | 98.1 |
| Val | 99.2 |

The fluctuation band here is about 1%. The percentage values indicate only the ratio of fully ¹³C labeled (U-¹³C) amino acids to fully unlabeled (U-¹²C) amino acids. Amino acids which have been only partially marked, are not included in the type of experiment.

### Additional information

### Reversed Phase Liquid Chromatography Separation (RPLC)

The RP-separation was carried out on the silica based column Acquity from Waters with a length of 15 cm and a particle size of 1.8 µm. The column oven temperature was 40°C. As eluent A 99.9% LC-MS grade water with 0.1 % formic acid and as eluent B 100% LC-MS grade methanol was used. The injection volume was 5 µl and the flow rate was set at 0.300 ml/min with an overall runtime of 12 min. The gradient was set as follows:

**Gradient RP-LC:**

| **Number** | **Eluent A [%]** | **Eluent B [%]** | **Time [min]** | **Flow rate [mL/min]** |
|---|---|---|---|---|
| 1 | 100 | 0 | 2.00 | 0.300 |
| 2 | 60 | 40 | 5.00 | 0.300 |
| 3 | 60 | 40 | 5.70 | 0.300 |
| 4 | 0 | 100 | 5.80 | 0.300 |
| 5 | 0 | 100 | 7.80 | 0.300 |
| 6 | 100 | 0 | 7.90 | 0.300 |
| 7 | 100 | 0 | 12.00 | 0.300 |

### Mass Spectrometry

The LC was connected to a triple quadrupole-mass spectrometer with ESI ionisation in positive ion mode and operated in the dynamic MRM mode to obtain a high sensitivity. The transitions for each amino acid and their corresponding standard, the retention times and mass transitions have been taken from an already existing method and optimized. Therefore, analysis in MRM mode was carried out to check and optimize retention times and mass transitions and then the parameters were applied on the dynamic MRM method.

One of the mass transitions of each amino acid was then used for quantification and one was used for qualification. In general there have been two transitions for every substance and the more intense one was taken as quantifier. However, for ¹³C proline there could be measured only one product ion. For identification of amino acids with the same mass of product and precursor ion the different retention time made the identification possible. The signal for glycine was weak due to in-source fragmentation, so in addition to one mass transition the mass of glycine was measured both as precursor and product ion to get a more intense signal. In-source fragmentation occurred at leucine and isoleucine, but it was not a problem for identification or quantification as they have different retention times and therefore could be distinguished by that.

The ESI-source was operated in positive ion mode and further configurations were as follows:

**ESI-parameters:**

| **Parameter** | **Setting** |
|---|---|
| Gas Temperature [°C] | 130 |
| Gas Flow [L/min] | 14 |
| Nebulizer [psi] | 40 |
| Sheath Gas Temperature [°C] | 360 |
| Sheath Gas Flow [L/min] | 12 |
| Ion modus | Positive |
| Capillary [V] | 3000 |
| Nozzle Voltage [V] | 500 |
| High Pressure RF [V] | 90 |
| Low Pressure RF [V] | 60 |

The following shows the mass transitions, collision energy, retention times and time windows after RP separation:

**Parameters for the dynamic MRM mode after RP separation:**

| **Compound** | **Precursor Ion** | **Product Ion** | **Coll E. [V]** | **Ret. time [min]** | **Time Window [min]** |
|---|---|---|---|---|---|
| **Ala** | 90.05 | 44.1 | 8 | 1.2 | 0.8 |
| **C13 Ala** | 93 | 46 | 8 | 1.2 | 0.8 |
| **Arg** | 175.11 | 116.1 | 12 | 1.14 | 1 |
| **Arg** | 175.11 | 70.2 | 36 | 1.14 | 1 |
| **C13 Arg** | 181 | 121 | 12 | 1.14 | 1 |
| **C13 Arg** | 181 | 74 | 36 | 1.14 | 1 |
| **Asn** | 133.05 | 87.1 | 8 | 1.2 | 1 |
| **Asn** | 133.05 | 74 | 12 | 1.2 | 1 |
| **C13 Asn** | 137 | 90 | 8 | 1.2 | 1 |
| **C13 Asn** | 137 | 76 | 12 | 1.2 | 1 |
| **Asp** | 134.04 | 88 | 8 | 1.2 | 1 |
| **Asp** | 134.04 | 74 | 12 | 1.2 | 1 |
| **C13 Asp** | 138 | 91 | 8 | 1.2 | 1 |
| **C13 Asp** | 138 | 76 | 12 | 1.2 | 1 |
| **Cys** | 122.02 | 76.1 | 8 | 1.33 | 0.9 |
| **Cys** | 122.02 | 59 | 32 | 1.33 | 0.9 |
| **Cys** | 122.02 | 58 | 40 | 1.33 | 0.9 |
| **C13 Cys** | 125 | 78.1 | 8 | 1.33 | 1 |
| **C13 Cys** | 125 | 61 | 32 | 1.33 | 1 |
| **Cystine** | 241 | 151.55 | 20 | 1.1 | 3 |
| **Cystine** | 241 | 74.05 | 16 | 1.1 | 3 |
| **C13 Cystine** | 246.8 | 154 | 20 | 1.1 | 3 |
| **C13 Cystine** | 246.8 | 76.05 | 16 | 1.1 | 3 |
| **Gin** | 147.07 | 130.1 | 8 | 1.22 | 0.93 |
| **Gin** | 147.07 | 84.1 | 16 | 1.22 | 0.93 |
| **C13 Gin** | 152 | 135 | 8 | 1.22 | 0.93 |
| **C13 Gin** | 152 | 88 | 16 | 1.22 | 0.93 |
| **Glu** | 148.05 | 130 | 4 | 1.3 | 0.9 |
| **Glu** | 148.05 | 84.1 | 12 | 1.3 | 0.9 |
| **C13 Glu** | 153 | 135 | 4 | 1.3 | 0.9 |
| **C13 Glu** | 153 | 88 | 12 | 1.3 | 0.9 |
| **Gly** | 76.03 | 76.03 | 0 | 1.3 | 3 |
| **Gly** | 76.03 | 59.1 | 12 | 1.3 | 3 |
| **C13 Gly** | 78.03 | 78.03 | 0 | 1.3 | 3 |
| **C13 Gly** | 78.03 | 60.03 | 12 | 1.3 | 3 |
| **His** | 156.07 | 110 | 12 | 1.12 | 0.71 |
| **His** | 156.07 | 83 | 28 | 1.12 | 0.71 |
| **C13 His** | 162 | 135 | 12 | 1.12 | 0.71 |
| **C13 His** | 162 | 88 | 28 | 1.12 | 0.71 |
| **Ile** | 132.09 | 86.1 | 8 | 4.6 | 1 |
| **Ile** | 132.09 | 69 | 25 | 4.6 | 1 |
| **Ile** | 86 | 57 | 20 | 4.6 | 1 |
| **C13 Ile** | 138 | 91 | 8 | 4.6 | 1 |
| **C13 Ile** | 91 | 60 | 20 | 4.6 | 1 |
| **Leu** | 132.1 | 86 | 8 | 4.75 | 1 |
| **Leu** | 86 | 43 | 20 | 4.75 | 1 |
| **C13 Leu** | 138 | 91 | 8 | 4.75 | 1 |
| **C13 Leu** | 91 | 46 | 20 | 4.75 | 1 |
| **Lys** | 147.11 | 130.1 | 8 | 1 | 2 |
| **Lys** | 147.11 | 84.1 | 16 | 1 | 2 |
| **C13 Lys** | 153 | 136 | 8 | 1 | 2 |
| **C13 Lys** | 153 | 89 | 16 | 1 | 2 |
| **Met** | 150.05 | 104.1 | 8 | 2.7 | 1 |
| **Met** | 150.05 | 55.9 | 12 | 2.7 | 1 |
| **C13 Met** | 155 | 108.9 | 8 | 2.7 | 1 |
| **C13 Met** | 155 | 59 | 12 | 2.7 | 1 |
| **Phe** | 166.08 | 120.1 | 12 | 5.68 | 1 |
| **Phe** | 166.08 | 77.2 | 40 | 5.68 | 1 |
| **C13 Phe** | 175 | 128 | 12 | 5.68 | 1 |
| **C13 Phe** | 175 | 83.15 | 40 | 5.68 | 1 |
| **Pro** | 116.06 | 70.1 | 16 | 1.51 | 1 |
| **Pro** | 116.06 | 43 | 36 | 1.51 | 1 |
| **C13 Pro** | 121 | 74 | 16 | 1.51 | 1 |
| **Ser** | 106.04 | 88 | 4 | 1.16 | 2 |
| **Ser** | 106.04 | 60.1 | 12 | 1.16 | 2 |
| **C13 Ser** | 109 | 91 | 4 | 1.16 | 2 |
| **C13 Ser** | 109 | 62 | 12 | 1.16 | 2 |
| **Thr** | 120.07 | 102.07 | 4 | 1.25 | 1.5 |
| **Thr** | 120.07 | 74.1 | 8 | 1.25 | 1.5 |
| **Thr** | 120.07 | 56.1 | 16 | 1.25 | 1.5 |
| **C13 Thr** | 124 | 77 | 8 | 1.25 | 1.5 |
| **C13 Thr** | 124 | 59 | 16 | 1.25 | 1.5 |
| **Trp** | 205.09 | 188 | 16 | 6.35 | 1 |
| **Trp** | 205.09 | 148.1 | 7 | 6.35 | 1 |
| **C13 Trp** | 216 | 199 | 16 | 6.35 | 1 |
| **C13 Trp** | 216 | 155 | 7 | 6.35 | 1 |
| **Tyr** | 182.07 | 136 | 12 | 4.6 | 1 |
| **Tyr** | 182.07 | 123.1 | 16 | 4.6 | 1 |
| **C13 Tyr** | 191 | 144 | 12 | 4.6 | 1 |
| **C13 Tyr** | 191 | 98 | 28 | 4.6 | 1 |
| **Val** | 118.08 | 72.1 | 8 | 2.17 | 1.01 |
| **Val** | 118.08 | 55 | 24 | 2.17 | 1.01 |
| **C13 Val** | 123 | 76 | 8 | 2.17 | 1.01 |
| **C13 Val** | 123 | 59 | 24 | 2.17 | 1.01 |

### Example 2: Preparation of uniformly ¹³C-labeled lipids from yeast cell wall

### Reagents

99% ¹³C₆-glucose (Sigma Aldrich, Steinheim, Germany), acid washed glass beads (425-600 µm particle size), chloroform, ammonium bicarbonate (ABC), ammonium acetate (NH₄Ac) and ammonium hydroxide (NH₄OH) were acquired from Sigma Aldrich (Steinheim, Germany). Water with 0.1% ammonium acetate (LC-MS grade) was purchased from Fluka (Buchs, Switzerland). Acetonitrile, 2-propanol and methanol (UPLC-grade) were obtained from Biosolve (Valkenswaard, The Netherlands).

### Production of Pichia pastoris cell walls

*Pichia pastoris* cells were grown on 99% ¹³C₆-glucose (Sigma Aldrich, Steinheim, Germany) as sole carbon source to produce a uniformly ¹³C-labeled yeast metabolome. Labeling efficiency was improved by cultivating yeast precultures in shaker flasks prior to the actual inoculation of the fermentor. Fed-batch fermentation was performed for 72 h utilizing a BioFlo 310 Fermenter (New Brunswick™). The cells were quenched in 60% cold methanol and the soluble cytosolic and insoluble cell wall containing fraction was extracted in 75% boiling ethanol for 3 min at 85°C prior to centrifugation at 4000×g and at -20°C for 10 min according to Neubauer S et al., J Sep Sci, 2012, 35(22):3091-105. The insoluble cell wall containing fraction corresponding to 1 L of fermentation broth and approximately 400 billion cells was collected and stored at -80°C until further processing.

### Lipid extraction from Pichia pastoris cell walls

20 mL 150 mM ammoniumbicarbonate buffer was added to the collected cell wall pellets at 4°C and the samples were mixed thoroughly. 500 µL of the solution was added to acid washed beads corresponding to 200 µL volume and mechanical disruption 3x 10 sec at highest frequency and cooling 30 sec on ice in between each round of disruption to make the cell wall material more accessible. The samples were mixed and 50 µL aliquots of the supernatant were used for Folch extraction with 990 µL chloroform/methanol solution (2:1 v/v) and incubated for 1 h at 4°C and 1400 rpm. After 1 h, 248 µL 150 mM ammonium bicarbonate buffer was added to all samples to induce phase separation. The samples were mixed and centrifuged 10 min at 10000 rcf at 4°C. The upper phase was removed and the lower lipid containing phase was evaporated with nitrogen and stored at -80°C until further analysis.

### Lipid analysis by shotgun MS

50 µL of isopropanol/methanol/chloroform (4:2:1) containing 7.5 mM ammonium formate was added to dissolve the dried lipids. The sample were transferred into a 96 well plate (Eppendorf, Hamburg, Germany) and then infused via robotic nanoflow ion source TriVersa NanoMate (Advion BioSciences, Ithaca NY, USA) into a Velos Orbitrap instrument (Thermo Fisher Scientific, Bremen, Germany) using chips with spraying nozzles of 4.1 µn. Shotgun parameters were applied as previously described (Herzog R et al., PLoS One, 2012, 7(1):e29851).

### Isotope enrichment determination

The isotope enrichment was assessed by using high resolution mass spectrometry (Velos Orbitrap instrument, Thermo Fisher Scientific, Bremen, Germany). Theoretical isotopologue distributions for different ¹³C enrichment degrees were calculated using Isopro software (Version 3.1) fitted to the experimental data.

### Qualitative assessment of the ¹³C labeled cell wall lipids

The resulting cell wall lipid profiles observed were similar to lipids extracted from whole *Pichia pastoris* cells, only that some lipid classes such ergosterol, phosphatidyl serine, cardiolipin, dimyristoylphosphoethanolamine, inositolphosphorylceramide and mannosylinositolphosphorylceramide were enriched, whereas ceramides and hexosyl ceramides decreased. The especially pronounced increase detected in the classes of ergosterol, phosphatidyl serine and dimyristoylphosphoethanolamine and decrease in ceramides is consistent with literature comparing cell homogenate and cell wall lipid fractions obtained from *Pichia pastoris* (Grillitsch K et al., Biochim Biophys Acta, 2014, 1838(7): 1889-97). Overall, approximately 250 lipid species were identified in *Pichia pastoris* cell wall and the ¹³C enrichment of the purified lipids was between 99.5-99.8%.

¹³C labeled lipids identified in *Pichia pastoris* cell walls using shotgun high resolution MS analysis in both negative and positive mode:

| **Lipid classes** | **Group** | **MS mode** |
|---|---|---|
| Fatty acids (FA) | Saturated fatty acids | - |
| | Unsaturated fatty acids | - |
| Glycerophospholipids (GP) | Phosphatidyl acid (PA) | - |
| | Phosphatidylethanolamine (PE) | - |
| | Phosphatidylcholine (PC) | - |
| | Phosphatidylglycerols (PG) | - |
| | Phosphatidylinositol (PI) | - |
| | Phosphatidylserine (PS) | - |
| | Lysophospholipids (LysoGPLs) | - |
| | Cardiolipin (CL) | - |
| | Dimyristoylphosphoethanolamine (DMPE) | + |
| Glycerolipids (GL) | Diacylglycerol (DAG) | + |
| | Triacylglycerol (TAG) | + |
| Sphingolipids (SL) | Ceramides (Cer) | + |
| | Hexosyl ceramide (HexCer) | + |
| | Inositolphosphorylceramides (IPC) | - |
| | Mannosylinositolphosphorylceramides (MIPC) | - |
| Sterols (ST) | Ergosterols | + |

Lipid species identified in the yeast cell wall with their representative monoisotopic uniformly labeled ¹³C mass and sum formula:

| **Number** | **Lipid species** | **U¹³C mass** | **Sum formula of detected adduct** |
|---|---|---|---|
| 1 | FA [18:0] | 301.324095 | C18 H35 O2 |
| 2 | FA [18:1] | 299.308445 | C18 H33 O2 |
| 3 | FA [18:2] | 297.292795 | C18 H31 O2 |
| 4 | FA [18:3] | 295.277145 | C18 H32 O2 |
| 5 | FA [18:4] | 293.261495 | C18 H29 O2 |
| 6 | FA [16:0] | 271.286085 | C16 H31 O2 |
| 7 | FA [16:1] | 269.270435 | C16 H29 O2 |
| 8 | FA [16:2] | 267.254785 | C16 H27 O2 |
| 9 | PA [32:2] | 678.5513 | H64 C35 08 P1 |
| 10 | PA [32:1] | 680.5670 | H66 C35 O8 P1 |
| 11 | PA [33:3] | 691.5547 | H64 C36 O8 P1 |
| 12 | PA [33:2] | 693.5703 | H66 C36 O8 P1 |
| 13 | PA [33:1] | 695.5860 | H68 C36 O8 P1 |
| 14 | PA [34:4] | 704.5580 | H64 C37 O8 P1 |
| 15 | PA [34:3] | 706.5737 | H66 C37 O8 P1 |
| 16 | PA [34:2] | 708.5893 | H68 C37 O8 P1 |
| 17 | PA [34:1] | 710.6050 | H70 C37 O8 P1 |
| 18 | PA [35:4] | 719.5770 | H66 C38 O8 P1 |
| 19 | PA [35:3] | 721.5927 | H68 C38 O8 P1 |
| 20 | PA [35:2] | 723.6083 | H70 C38 O8 P1 |
| 21 | PA [35:1] | 725.6240 | H72 C38 O8 P1 |
| 22 | PA [36:6] | 730.5647 | H64 C39 O8 P1 |
| 23 | PA [36:5] | 732.5804 | H66 C39 O8 P1 |
| 24 | PA [36:4] | 734.5960 | H68 C39 O8 P1 |
| 25 | PA [36:3] | 736.6117 | H70 C39 O8 P1 |
| 26 | PA [36:2] | 738.6273 | H72 C39 O8 P1 |
| 27 | PA [36:1] | 740.6430 | H74 C39 O8 P1 |
| 28 | PC [32:3] | 812.6471 | H75 C41 N1 O10 P1 |
| 29 | PC [32:2] | 814.6627 | H77 C41 N1 O10 P1 |
| 30 | PC [32:1] | 816.6784 | H79 C41 N1 O10 P1 |
| 31 | PC [33:4] | 825.6504 | H75 C42 N1 O10 P1 |
| 32 | PC [33:3] | 827.6661 | H77 C42 N1 O10 P1 |
| 33 | PC [33:2] | 829.6817 | H79 C42 N1 O10 P1 |
| 34 | PC [33:1] | 831.6974 | H81 C42 N1 O10 P1 |
| 35 | PC [34:6] | 836.6381 | H73 C43 N1 O10 P1 |
| 36 | PC [34:5] | 838.6538 | H75 C43 N1 O10 P1 |
| 37 | PC [34:4] | 840.6694 | H77 C43 N1 O10 P1 |
| 38 | PC [34:3] | 842.6851 | H79 C43 N1 O10 P1 |
| 39 | PC [34:2] | 844.7007 | H81 C43 N1 O10 P1 |
| 40 | PC [34:1] | 846.7164 | H83 C43 N1 O10 P1 |
| 41 | PC [35:6] | 851.6571 | H75 C44 N1 O10 P1 |
| 42 | PC [35:5] | 853.6728 | H77 C44 N1 O10 P1 |
| 43 | PC [35:4] | 855.6884 | H79 C44 N1 O10 P1 |
| 44 | PC [35:3] | 857.7041 | H81 C44 N1 O10 P1 |
| 45 | PC [35:2] | 859.7197 | H83 C44 N1 O10 P1 |
| 46 | PC [35:1] | 861.7354 | H85 C44 N1 O10 P1 |
| 47 | PC [36:7] | 866.6761 | H87 C45 N1 O10 P1 |
| 48 | PC [36:5] | 868.6918 | H87 C45 N1 O10 P1 |
| 49 | PC [36:4] | 870.7074 | H87 C45 N1 O10 P1 |
| 50 | PC [36:3] | 872.7231 | H87 C45 N1 O10 P1 |
| 51 | PC [36:2] | 874.7387 | H87 C45 N1 O10 P1 |
| 52 | PC [36:1] | 876.7544 | H87 C45 N1 O10 P1 |
| 53 | PC [37:4] | 885.7264 | H89 C46 N1 O10 P1 |
| 54 | PC [37:3] | 887.7421 | H89 C46 N1 O10 P1 |
| 55 | PC [37:2] | 889.7577 | H89 C46 N1 O10 P1 |
| 56 | PC [38:4] | 898.7298 | H85 C47 N1 O10 P1 |
| 57 | PC [38:3] | 900.7454 | H87 C47 N1 O10 P1 |
| 58 | PC [38:2] | 902.7611 | H89 C47 N1 O10 P1 |
| 59 | PC [38:1] | 904.7767 | H91 C47 N1 O10 P1 |
| 60 | PE [32:3] | 721.5846 | H67 C37 N1 O8 P1 |
| 61 | PE [32:2] | 723.6002 | H69 C37 N1 O8 P1 |
| 62 | PE [32:1] | 725.6159 | H71 C37 N1 O8 P1 |
| 63 | PE [33:2] | 738.6192 | H71 C38 N1 O8 P1 |
| 64 | PE [33:1] | 740.6349 | H73 C38 N1 O8 P1 |
| 65 | PE [34:5] | 747.5913 | H67 C39 N1 O8 P1 |
| 66 | PE [34:4] | 749.6069 | H69 C39 N1 O8 P1 |
| 67 | PE [34:3] | 751.6226 | H71 C39 N1 O8 P1 |
| 68 | PE [34:2] | 753.6382 | H73 C39 N1 O8 P1 |
| 69 | PE [34:1] | 755.6539 | H75 C39 N1 O8 P1 |
| 70 | PE [35:6] | 760.5946 | H67 C40 N1 O8 P1 |
| 71 | PE [35:5] | 762.6103 | H69 C40 N1 O8 P1 |
| 72 | PE [35:4] | 764.6259 | H71 C40 N1 O8 P1 |
| 73 | PE [35:3] | 766.6416 | H73 C40 N1 O8 P1 |
| 74 | PE [35:2] | 768.6572 | H75 C40 N1 O8 P1 |
| 75 | PE [35:1] | 770.6729 | H77 C40 N1 O8 P1 |
| 76 | PE [36:6] | 775.6136 | H69 C41 N1 O8 P1 |
| 77 | PE [36:5] | 777.6293 | H71 C41 N1 O8 P1 |
| 78 | PE [36:4] | 779.6449 | H73 C41 N1 O8 P1 |
| 79 | PE [36:3] | 781.6606 | H75 C41 N1 O8 P1 |
| 80 | PE [36:2] | 783.6762 | H77 C41 N1 O8 P1 |
| 81 | PE [36:1] | 785.6919 | H79 C41 N1 O8 P1 |
| 82 | PG [32:1] | 757.6138 | H72 C38 O10 P1 |
| 83 | PG [33:1] | 772.6328 | H74 C39 O10 P1 |
| 84 | PG [34:4] | 781.6049 | H70 C40 O10 P1 |
| 85 | PG [34:3] | 783.6205 | H72 C40 O10 P1 |
| 86 | PG [34:2] | 785.6362 | H74 C40 O10 P1 |
| 87 | PG [34:1] | 787.6518 | H76 C40 O10 P1 |
| 88 | PG [36:6] | 807.6116 | H70 C42 O10 P1 |
| 89 | PG [36:5] | 809.6272 | H72 C42 O10 P1 |
| 90 | PG [36:4] | 811.6429 | H74 C42 O10 P1 |
| 91 | PG [36:3] | 813.6585 | H76 C42 O10 P1 |
| 92 | PG [36:2] | 815.6742 | H78 C42 O10 P1 |
| 93 | PG [36:1] | 817.6898 | H80 C42 O10 P1 |
| 94 | PI [32:2] | 846.6253 | H74 C41 O13 P1 |
| 95 | PI [32:1] | 848.6412 | H76 C41 O13 P1 |
| 96 | PI [33:2] | 861.6456 | H76 C42 O13 P1 |
| 97 | PI [33:1] | 863.6606 | H78 C42 O13 P1 |
| 98 | PI [34:3] | 874.6487 | H76 C43 O13 P1 |
| 99 | PI [34:2] | 876.6644 | H78 C43 O13 P1 |
| 100 | PI [34:1] | 878.6807 | H80 C43 O13 P1 |
| 101 | PI [35:2] | 891.6824 | H80 C44 O13 P1 |
| 102 | PI [35:1] | 893.6995 | H82 C44 O13 P1 |
| 103 | PI [36:6] | 898.6388 | H74 C45 O13 P1 |
| 104 | PI [36:5] | 900.6549 | H76 C45 O13 P1 |
| 105 | PI [36:4] | 902.6712 | H78 C45 O13 P1 |
| 106 | PI [36:3] | 904.6874 | H80 C45 O13 P1 |
| 107 | PI [36:2] | 906.7012 | H82 C45 O13 P1 |
| 108 | PI [36:1] | 908.7179 | H84 C45 O13 P1 |
| 109 | PS [32:2] | 768.5962 | H69 C38 N1 O10 P1 |
| 110 | PS [32:1] | 770.6104 | H71 C38 N1 O10 P1 |
| 111 | PS [34:3] | 796.6173 | H71 C40 N1 O10 P1 |
| 112 | PS [34:2] | 798.6329 | H73 C40 N1 O10 P1 |
| 113 | PS [34:1] | 800.6493 | H75 C40 N1 O10 P1 |
| 114 | PS [36:5] | 822.6242 | H71 C42 N1 O10 P1 |
| 115 | PS [36:4] | 824.6395 | H73 C42 N1 O10 P1 |
| 116 | PS [36:3] | 826.6558 | H75 C42 N1 O10 P1 |
| 117 | PS [36:2] | 828.6700 | H77 C42 N1 O10 P1 |
| 118 | PS [36:1] | 830.6865 | H79 C42 N1 O10 P1 |
| 119 | LPA [16:0] | 428.2993 | H38 C19 O7 P1 |
| 120 | LPA [18:2] | 454.3060 | H39 C21 O7 P1 |
| 121 | LPA [18:1] | 456.3216 | H41 C21 O7 P1 |
| 122 | LPE [15:0] | 458.3292 | H41 C20 N1 O7 P1 |
| 123 | LPE [16:1] | 471.3325 | H41 C21 N1 O7 P1 |
| 124 | LPE [16:0] | 473.3482 | H43 C21 N1 O7 P1 |
| 125 | LPE [17:3] | 482.3202 | H39 C22 N1 O7 P1 |
| 126 | LPE [17:2] | 484.3359 | H41 C22 N1 O7 P1 |
| 127 | LPE [17:1] | 486.3515 | H43 C22 N1 O7 P1 |
| 128 | LPE [18:1] | 501.3705 | H45 C23 N1 O7 P1 |
| 129 | LPE [18:0] | 503.3862 | H47 C23 N1 O7 P1 |
| 130 | LPG [16:0] | 505.3461 | H44C22 O9 P1 |
| 131 | LPG [18:3] | 529.3372 | H42 C24 O9 P2 |
| 132 | LPG [18:2] | 531.3528 | H44 C24 O9 P1 |
| 133 | LPG [18:1] | 533.3685 | H46 C24 O9 P1 |
| 134 | LPC [16:2] | 560.3794 | H49 C26 N1 O9 P1 |
| 135 | LPC [16:1] | 562.3950 | H51 C26 N1 O9 P1 |
| 136 | LPC [16:0] | 564.4107 | H53 C26 N1 O9 P1 |
| 137 | LPC [18:0] | 610.4398 | H57 C28 N1 O9 P1 |
| 138 | IPC [34:0,2] | 820.6738 | H79 C40 N1 O11 P1 |
| 139 | IPC [36:0,2] | 850.7118 | H83 C42 N1 O11 P1 |
| 140 | IPC [36:0,4] | 882.7017 | H83 C42 N1 O13 P1 |
| 141 | IPC [42:0,4] | 972.8157 | H95 C48 N1 O13 P1 |
| 142 | IPC [42:0,5] | 988.8106 | H95 C48 N1 O14 P1 |
| 143 | IPC [44:0,4] | 1002.8537 | H99 C50 N1 O13 P1 |
| 144 | IPC [44:0,5] | 1018.8486 | H99 C50 N1 O14 P1 |
| 145 | MIPC [42:0,4] | 1140.8886 | H105 C54 O18 N1 P1 |
| 146 | MIPC [42:0,5] | 1156.8836 | H105 C54 O19 N1 P1 |
| 147 | MIPC [44:0,4] | 1170.9267 | H109 C56 O18 N1 P1 |
| 148 | MIPC [44:0,5] | 1186.9216 | H109 C56 O19 N1 P1 |
| 149 | CL [70:6] | 1503.2300 | H141 C79 O17 P2 |
| 150 | CL [71:9] | 1512.2020 | H 137 C80 O17 P2 |
| 151 | CL [71:8] | 1514.2177 | H139 C80 O17 P2 |
| 152 | CL [72:11] | 1523.1897 | H135 C81 O17 P2 |
| 153 | CL [72:10] | 1525.2054 | H137 C81 O17 P2 |
| 154 | CL [72:9] | 1527.2210 | H139 C81 O17 P2 |
| 155 | CL [72:8] | 1529.2367 | H141 C81 O17 P2 |
| 156 | CL [72:7] | 1531.2523 | H143 C81 O17 P2 |
| 157 | CL [72:6] | 1533.2680 | H145 C81 O17 P2 |
| 158 | CL [72:5] | 1535.2836 | H147 C81 O17 P2 |
| 159 | DMPE [36:4] | 781.6601 | H75 C41 N1 O8 P1 |
| 160 | DMPE [36:3] | 783.6770 | H77 C41 N1 O8 P1 |
| 161 | DMPE [36:2] | 785.6921 | H79 C41 N1 O8 P1 |
| 162 | DMPE [36:1] | 787.7082 | H81 C41 N1 O8 P1 |
| 163 | DMPE [38:6] | 805.6590 | H75 C43 N1 O8 P1 |
| 164 | DMPE [38:5] | 807.6752 | H77 C43 N1 O8 P1 |
| 165 | DMPE [38:4] | 809.6916 | H79 C43 N1 O8 P1 |
| 166 | DAG [32:2] | 617.6272 | H78 C35 O5 N1 |
| 167 | DAG [32:1] | 619.6428 | H70 C35 O5 N1 |
| 168 | DAG [33:2] | 632.6462 | H80 C36 O5 N1 |
| 169 | DAG [34:4] | 643.6339 | H78 C37 O5 N1 |
| 170 | DAG [34:3] | 645.6495 | H80 C37 O5 N1 |
| 171 | DAG [34:2] | 647.6652 | H82 C37 O5 N1 |
| 172 | DAG [34:1] | 649.6808 | H84 C37 O5 N1 |
| 173 | DAG [35:6] | 654.6216 | H86 C38 O5 N1 |
| 174 | DAG [35:5] | 656.6372 | H80 C38 O5 N1 |
| 175 | DAG [35:4] | 658.6529 | H80 C38 O5 N1 |
| 176 | DAG [35:3] | 660.6685 | H82 C38 O5 N1 |
| 177 | DAG [35:2] | 662.6842 | H84 C38 O5 N1 |
| 178 | DAG [35:1] | 664.6998 | H86 C38 O5 N1 |
| 179 | DAG [36:7] | 667.6249 | H76 C39 O5 N1 |
| 180 | DAG [36:6] | 669.6406 | H78 C39 O5 N1 |
| 181 | DAG [36:5] | 671.6562 | H80 C39 O5 N |
| 182 | DAG [36:4] | 673.6719 | H82 C39 O5 N1 |
| 183 | DAG [36:3] | 675.6875 | H84 C39 O5 N1 |
| 184 | DAG [36:2] | 677.7032 | H86 C39 O5 N1 |
| 185 | DAG [36:1] | 679.7188 | H88 C39 O5 N1 |
| 186 | DAG [38:7] | 697.6630 | H80 C41 O5 N1 |
| 187 | DAG [38:6] | 699.6786 | H82 C41 O5 N1 |
| 188 | DAG [38:5] | 701.6943 | H84 C41 O5 N1 |
| 189 | DAG [38:4] | 703.7099 | H86 C41 O5 N1 |
| 190 | DAG [38:3] | 705.7256 | H88 C41 O5 N1 |
| 191 | DAG [38:2] | 707.7412 | H90 C41 O5 N1 |
| 192 | TAG [50:6] | 893.8854 | H94 C53 O6 N1 |
| 193 | TAG [50:5] | 895.9010 | H96 C53 O6 N1 |
| 194 | TAG [50:4] | 897.9167 | H98 C53 O6 N1 |
| 195 | TAG [50:3] | 899.9323 | H100 C53 O6 N1 |
| 196 | TAG [50:2] | 901.9480 | H102 C53 O6 N1 |
| 197 | TAG [51:6] | 908.9044 | H96 C54 O6 N1 |
| 198 | TAG [51:5] | 910.9200 | H98 C54 O6 N1 |
| 199 | TAG [51:4] | 912.9357 | H100 C54 O6 N1 |
| 200 | TAG [51:3] | 914.9513 | H102 C54 O6 N1 |
| 201 | TAG [51:2] | 916.9670 | H104 C54 O6 N1 |
| 202 | TAG [52:6] | 923.9234 | H98 C55 O6 N1 |
| 203 | TAG [52:5] | 925.9390 | H100 C55 O6 N1 |
| 204 | TAG [52:4] | 927.9547 | H102 C55 O6 N1 |
| 205 | TAG [52:3] | 929.9703 | H104 C55 O6 N1 |
| 206 | TAG [52:2] | 931.9860 | H106 C55 O6 N1 |
| 207 | TAG [53:4] | 942.9737 | H104 C56 O6 N1 |
| 208 | TAG [53:3] | 944.9893 | H106 C56 O6 N1 |
| 209 | TAG [54:7] | 951.9458 | H100 C57 O6 N1 |
| 210 | TAG [54:6] | 953.9614 | H102 C57 O6 N1 |
| 211 | TAG [54:5] | 955.9771 | H104 C57 O6 N1 |
| 212 | TAG [54:4] | 957.9927 | H106 C57 O6 N1 |
| 213 | TAG [54:3] | 960.0084 | H108 C57 O6 N1 |
| 214 | TAG [54:2] | 962.0240 | H110 C57 O6 N1 |
| 215 | TAG [56:5] | 986.0151 | H106 C58 O6 N1 |
| 216 | TAG [56:4] | 988.0307 | H108 C58 O6 N1 |
| 217 | TAG [56:3] | 990.0464 | H110 C58 O6 N1 |
| 218 | Erg[16:0] | 696.7509 | H78 C44 O2 N1 |
| 219 | Erg[16:1] | 694.7352 | H76 C44 O2 N1 |
| 220 | Erg[16:2] | 692.7196 | H74 C44 O2 N1 |
| 221 | Erg[17:1] | 709.754229 | H78 C45 O2 N1 |
| 222 | Erg[18:0] | 726.788884 | H82 C46 O2 N1 |
| 223 | Erg[18:1] | 724.773234 | H80 C46 O2 N1 |
| 224 | Erg[18:2] | 722.757584 | H78 C46 O2 N1 |
| 225 | Erg[18:3] | 720.741934 | H76 C46 O2 N1 |
| 226 | Erg[19:2] | 737.776589 | H80 C47 O2 N1 |
| 227 | Erg[19:3] | 735.760939 | H78 C47 O2 N1 |
| 228 | Erg[20:0] | 756.826894 | H86 C48 O2 N1 |
| 229 | Cer [36:2] | 600.656349 | H70 C36 O3 N1 |
| 230 | Cer [36:1] | 602.671999 | H72 C36 O3 N1 |
| 231 | Cer [36:2] ox | 616.651264 | H70 C36 O4 N1 |
| 232 | Cer [36:1] ox | 618.666914 | H72 C36 O4 N1 |
| 233 | HexCer [35:2] | 799.7432 | H82 C43 O9 N1 |

### Example 3: Quantitative assessment of monosaccharide concentrations in yeast cell wall based on commercially available yeast cell wall products

### Reagents

99% ¹³C₆-glucose, acid washed glass beads (425-600 µm particle size), chloroform, ammonium bicarbonate (ABC), ammonium acetate (NH₄Ac), sulfuric acid (H₂SO₄), barium hydroxide (BaOH), monosaccharide standards (glucose, mannose, glucosamine, ribose, arabinose, xylose, galactose, fucose), anthranilic acid, were acquired from Sigma Aldrich (Steinheim, Germany).

### H₂SO₄ Hydrolysis and sugar derivatisation

750 µL of H₂SO₄ were added to 100 mg of commercially available cell wall samples originating from *Saccharomyces cerevisiae* and incubated at room temperature for 3 h. Then the slurry was diluted with 9.5 mL H₂O containing 10 mM fucose as internal standard and incubated for 4 h at 100°C. Afterwards the solution was transferred to a 50 or 100 mL falcon tube and washed with 5 mL H₂O. A saturated 72 g/L BaOH solution was added until the pH of the samples reached neutrality (pH=6-8). Then the white BaSO₄ precipitate was left overnight at 4°C. The following day, a pH electrode was used to check pH (pH=6-8). 1 mL of the clear supernatant was transferred to an Eppendorf tube and centrifuged for 5 min at 8000 rpm and 4°C. 50 µL of the samples were dried by Speed-Vac. 1 mL of 0.6% sodium acetate solution was prepared as well as 6 mg antranilic acid and 20 mg NaCNBH₄ diluted in 1 mL methanol containing 2.4% sodium acetate and 2% boric acid. Firstly, 10 µL of the 0.6% sodium acetate was used to dissolve the dried sample or dried standards (10 µL of a 10 mM sugar solution). Afterwards 10 µL of the methanol reaction solution was added and the samples were incubated for 45-60 min at 80°C (weigh the vials down). Finally, 980 µL of H₂O were added and the samples were used for RP-FLD detection.

### Monosaccharide analysis with RP-FLD

The samples were diluted 1:10 and measured with a Hypersii C18 reversed phase column and quantified using the internal standard fucose and an external calibration with different derivatised sugar standards (glucose, mannose, glucosamine, galactose, arabinose, ribose, xylose, fucose) using a fluorescence detection system.

### Conditions:

- 5 µm particle size
- 250 x 4 mm
- Flow rate: 1 mL min⁻¹
- Buffer A: 0.2% butylamine, 0.5% phosphoric acid (take 85% orthophosphoric acid), 1% tetrahydrofuran in water
- Buffer B: 50% acetonitrile in Buffer A
- Fluorescence: Extinction at 360 nm, Emission 425 nm
- Gradient elution:

| | | | | | | |
|---|---|---|---|---|---|---|
| Time | 0 | 17 | 23 | 29 | 30 | 35 |
| % B | 5 | 25 | 100 | 100 | 5 | 5 |

An exemplary chromatogram of the monosaccharides derivatized with anthranilic acid is shown in Figure 3.

The monosaccharide concentrations in the different *Saccharomyces cerevisiae* samples thus determined are shown in Figure 4.

In accordance to literature, mannan and glucan were the highest abundant species in commercially available yeast cell wall (Kath F et al., Angew Makromol Chem, 1999, 268(1):59-68). Moreover, ribose is highly present in sample 5 and arabinose is increased in sample 3. Differing pattern can be due to different fermentation conditions and strain behaviour.

### Example 4: Preparation of uniformly ¹³C-labeled monosaccharides from yeast cell wall

### Reagents

99% ¹³C₆-glucose, acid washed glass beads (425-600 µm particle size), chloroform, ammonium bicarbonate (ABC), ammonium acetate (NH₄Ac), sulfuric acid (H₂SO₄), barium hydroxide (BaOH) were acquired from Sigma Aldrich (Steinheim, Germany).

### Production of Pichia pastoris cell walls

*Pichia pastoris* cells were grown on 99% ¹³C₆-glucose (Sigma Aldrich, Steinheim, Germany) as sole carbon source to produce a uniformly ¹³C-labeled yeast metabolome. Labeling efficiency was improved by cultivating yeast precultures in shaker flasks prior to the actual inoculation of the fermentor. Fed-batch fermentation was performed for 72 h utilizing a BioFlo 310 Fermenter (New Brunswick™). The cells were quenched in 60% cold methanol and the soluble cytosolic and insoluble cell wall containing fraction was extracted in 75% boiling ethanol for 3 min at 85°C prior to centrifugation at 4000×g and at -20°C for 10 min according to Neubauer S et al., J Sep Sci, 2012, 35(22):3091-105. The insoluble cell wall containing fraction corresponding to 1 L of fermentation broth and approximately 400 billion cells was collected. The cell wall pellet was washed with water and centrifuged at 4000xg and at -20°C for 10 min. The cell wall containing solutions were frozen at -80°C for 1 h prior to drying by SpeedVac for approximately 5 h. Finally, the dried material was pulverized using a precleaned glass stick and stored at -20°C until further analysis.

### Lipid extraction

Aliquots of 20 mg dry ¹³C-labeled yeast cell wall were weighted in glass tubes and 4 mL of a 4:8:3 Chloroform:MeOH:H₂O were added to extract the lipids. Everything was placed for 15 min into a sonication bath to dissolve sample powder completely. Afterwards, the samples were shaken for 30 min at room temperature. The samples were put on ice for several minutes to decrease protein solubility in solvent. Then the samples were centrifuged for 10-15 min at 2500-3000 rpm at 4°C. The lipid containing supernatant was removed and the whole procedure was repeated. Finally, another 4 mL of the mixture was added prior to sample mixing, sonication and shaking over night at room temperature. The samples were cooled on ice, and the supernatant was removed after 10-15 min centrifugation at 2500-3000 rpm.

### H₂SO₄ Hydrolysis

750 µL of H₂SO₄ were added to 100 mg yeast cell wall sample and incubated at room temperature for 3 h. Then the slurry was diluted with 9.5 mL H₂O and incubated for 4 h at 100°C. Afterwards the solution was transferred to a 50 or 100 mL falcon tube and washed with 5 mL H₂O. A saturated 72 g/L BaOH solution was added until the pH of the samples reached neutrality (pH=6-8). Then the white BaSO₄ precipitate was left overnight at 4°C. The following day, a pH electrode was used to check pH (pH=6-8). 1 mL of the clear supernatant was transferred to an Eppendorf tube and centrifuged for 5 min at 8000 rpm and 4°C.

### ¹³C enrichment degree of monosaccharides in Pichia pastoris yeast cell wall

Monosaccharide carbon enrichment degree was assessed using the RP chromatography method presented in example 1 coupled to a high resolution TOF mass analyzer (Agilent 6230 TOF) in the positive mode. In example 3 the general monosaccharide species mannose, glucose, galactose, ribose, xylose and arabinose were identified and quantified in eight yeast cell wall products from *Saccharomyces cerevisiae.* It can be assumed that general monosaccharide classes are the same or at least similar in different yeast strains. Anthranilic acid derivatization (applied in example 3) changes the overall isotopic pattern, thus challenging appropriate ¹³C enrichment analysis via MS. As a compromise, RP-MS with coeluting monosaccharide species was applied in order to investigate the overall ¹³C enrichment in monosaccharides. Glucose, mannose, and galactose coeluted at 1.2 min (observed monoisotopic mass: ¹³C₆H₁₂O₆Na= 209.0727), ribose, xylose and arabinose eluted at 1.1 min (observed monoisotopic mass ¹³C₅H₁₀O₅Na: 178.0588) and glucosamine at 1.2 min (observed monoisotopic mass ¹³C₆H₁₄NO₅: 186.1068). All observed monosaccharides were 99.3-99.8% ¹³C enriched (enrichment degree was determined in the same manner as presented in example 2) and no unlabeled species were detected. These results prove that efficient ¹³C labeling is achieved for monosaccharides in yeast cell wall of *Pichia pastoris.*

## Claims

1. A method of preparing one or more stable isotope labeled metabolites selected from stable isotope labeled lipids, stable isotope labeled amino acids, stable isotope labeled monosaccharides, and mixtures thereof, the method comprising:
- cultivating a microorganism in the presence of a stable isotope labeled nutrient;
- isolating the cell wall and the cell membrane of the microorganism; and
- isolating said one or more stable isotope labeled metabolites from the isolated cell wall and/or the isolated cell membrane.

2. The method of claim 1, wherein said one or more stable isotope labeled metabolites are one or more stable isotope labeled lipids, and
wherein the step of isolating said one or more stable isotope labeled metabolites from the isolated cell wall and/or the isolated cell membrane is conducted by:
- extracting lipids from the isolated cell wall and/or the isolated cell membrane and thereby obtaining a mixture of stable isotope labeled lipids; and
- optionally, isolating one or more stable isotope labeled lipids from said mixture.

3. The method of claim 1, wherein said one or more stable isotope labeled metabolites are one or more stable isotope labeled amino acids and/or one or more stable isotope labeled monosaccharides, and
wherein the step of isolating said one or more stable isotope labeled metabolites from the isolated cell wall and/or the isolated cell membrane is conducted by:
- extracting peptides/proteins and/or polysaccharides from the isolated cell wall and/or the isolated cell membrane;
- hydrolyzing said peptides/proteins and/or said polysaccharides and thereby obtaining a mixture of stable isotope labeled amino acids and/or stable isotope labeled monosaccharides; and
- optionally, isolating one or more stable isotope labeled amino acids and/or one or more stable isotope labeled monosaccharides from said mixture.

4. The method of claim 1 or 3, wherein said one or more stable isotope labeled metabolites are one or more stable isotope labeled amino acids, and
wherein the step of isolating said one or more stable isotope labeled metabolites from the isolated cell wall and/or the isolated cell membrane is conducted by:
- extracting peptides and/or proteins from the isolated cell wall and/or the isolated cell membrane;
- hydrolyzing said peptides and/or proteins and thereby obtaining a mixture of stable isotope labeled amino acids; and
- optionally, isolating one or more stable isotope labeled amino acids from said mixture.

5. The method of claim 1 or 3, wherein said one or more stable isotope labeled metabolites are one or more stable isotope labeled monosaccharides, and
wherein the step of isolating said one or more stable isotope labeled metabolites from the isolated cell wall and/or the isolated cell membrane is conducted by:
- extracting polysaccharides from the isolated cell wall and/or the isolated cell membrane;
- hydrolyzing said polysaccharides and thereby obtaining a mixture of stable isotope labeled monosaccharides; and
- optionally, isolating one or more stable isotope labeled monosaccharides from said mixture.

6. The method of any one of claims 1 to 5, wherein the stable isotope is selected from ¹³C, ¹⁵N, ¹⁷O, ¹⁸O, ³³S ³⁴S and any combination thereof.

7. The method of any one of claims 1 to 6, wherein the stable isotope is ¹³C, optionally in combination with ¹⁵N and/or ³⁴S, and wherein the one or more stable isotope labeled metabolites are uniformly labeled with said stable isotope.

8. The method of any one of claims 1 to 7, wherein the stable isotope labeled lipid(s) is/are selected from fatty acids, glycerolipids, glycerophospholipids, sphingolipids, sterol lipids, prenol lipids, saccharolipids, and polyketides, all of which are stable isotope labeled,
wherein the stable isotope labeled amino acid(s) is/are selected from Ala, Arg, Asn, Asp, Cys, Glu, Gln, Gly, His, Ile, Leu, Lys, Met, Phe, Pro, Ser, Thr, Trp, Tyr, and Val, all of which are stable isotope labeled,
and wherein the stable isotope labeled monosaccharide(s) is/are selected from mannose, glucose, galactose, ribose, arabinose, xylose, rhamnose and N-acetylglucosamine, all of which are stable isotope labeled.

9. The method of any one of claims 1 to 8, wherein said microorganism is a yeast which is preferably a yeast of the genus *Pichia, Saccharomyces, Candida, Hansenula, Schizosaccharomyces,* or *Kluyveromyces,* and which is more preferably *Pichia pastoris.*

10. The method of any one of claims 1 to 9, wherein the step of isolating the cell wall and the cell membrane of the microorganism is conducted by: (i) boiling the cells of the microorganism in an ethanol/water mixture, in a methanol/water mixture or in a propanol/water mixture, and then isolating the cell wall and the cell membrane by filtration and/or centrifugation; or (ii) subjecting the cells of the microorganism to a freeze-thaw treatment, and then isolating the cell wall and the cell membrane by filtration and/or centrifugation; or (iii) subjecting the cells of the microorganism to sonication, and then isolating the cell wall and the cell membrane by filtration and/or centrifugation; or (iv) treating the cells of the microorganism with a detergent, and then isolating the cell wall and the cell membrane by filtration and/or centrifugation.

11. The method of any one of claims 1 to 10, wherein the step of isolating the cell wall and the cell membrane of the microorganism is conducted by boiling the cells of the microorganism in an ethanol/water mixture and filtering off the cell wall and the cell membrane.

12. The method of claim 2 or any one of its dependent claims 6 to 11, wherein the step of extracting lipids from the isolated cell wall and/or the isolated cell membrane is conducted using an organic solvent which is preferably selected from methyl-tert-butyl ether, chloroform/methanol, dichloromethane/methanol, hexane, hexane/isopropanol, acetone/ethanol, ethanol/diethyl ether, and diethyl ether.

13. The method of claim 3 or 4 or any one of their dependent claims 6 to 11, wherein the peptides and/or proteins are extracted from the isolated cell wall and/or the isolated cell membrane using one or more precipitation methods selected from chloroform/methanol precipitation, methyl-tert-butyl ether precipitation, diethyl ether precipitation, acetone precipitation, acidified acetone/methanol precipitation, trichloroacetic acid precipitation, and ammonium sulfate precipitation.

14. The method of claim 3 or 5 or any one of their dependent claims 6 to 11, wherein the step of extracting polysaccharides from the isolated cell wall and/or the isolated cell membrane is conducted using an organic solvent which is preferably selected from methyl-tert-butyl ether, chloroform/methanol, dichloromethane/methanol, hexane, hexane/isopropanol, acetone/ethanol, ethanol/diethyl ether, and diethyl ether.

15. A composition comprising a mixture of stable isotope labeled metabolites selected from stable isotope labeled lipids, stable isotope labeled amino acids and/or stable isotope labeled monosaccharides, which is obtainable by the method of any one of claims 1 to 14.
